# EUROPEAN PATENT SPECIFICATION

(11) **EP 3 318 632 B1**
(45) Date of publication and mention of the grant of the patent: **26.10.2022**
(21) Application number: 16818051.1
(22) Date of filing: 30.06.2016
(51) Int. Cl.: C12N 15/09, C12N 5/09, G01N 33/53, G01N 33/574, C12Q 1/6886

(54) **MARKER FOR HETEROGENEITY OF CANCER TISSUE, AND USE THEREOF**
MARKER DER HETEROGENITÄT VON KREBSGEWEBE UND VERWENDUNG DAVON
MARQUEUR DE L'HOMOGÉNÉITÉ D'UN TISSU CANCÉREUX ET UTILISATION DUDIT MARQUEUR

(30) Priority: 01.07.2015 JP 2015133033
(43) Date of publication of application: 09.05.2018
(73) Proprietor: Keio University, Tokyo 108-8345 (JP)
(72) Inventor: SAYA, Hideyuki, Tokyo 160-8582 (JP); ARIMA, Yoshimi, Tokyo 160-8582 (JP); SEMBA, Takashi, Tokyo 160-8582 (JP)
(74) Representative: Schnappauf, Georg
(86) International application number: PCT/JP2016/069529
(87) International publication number: WO 2017/002943

(56) References cited:
- WO-A1-2006/053442
- WO-A1-2010/064702
- WO-A1-2013/116144
- WO-A1-2014/144804
- CN-A- 101 993 944
- JP-A- 2007 527 220
- JP-A- 2009 513 161
- JP-A- 2012 525 159
- JP-A- 2013 507 987
- US-A1- 2014 100 121
- N. NAVIN ET AL: "Inferring tumor progression from genomic heterogeneity", GENOME RESEARCH, vol. 20, no. 1, 1 January 2010 (2010-01-01), pages 68-80, XP055210315, ISSN: 1088-9051, DOI: 10.1101/gr.099622.109
- LUCIANO G MARTELOTTO ET AL: "Breast cancer intra-tumor heterogeneity", BREAST CANCER RESEARCH, CURRENT MEDICINE GROUP LTD, GB, vol. 16, no. 3, 20 May 2014 (2014-05-20), page 210, XP021203826, ISSN: 1465-5411, DOI: 10.1186/BCR3658
- YANG, X. ET AL.: 'Microcirulatory fraction (MCF1) as a potential imaging marker for tumor heterogeneity in breast cancer.' MAGNETIC RESONANCE IMAGING vol. 30, no. 8, October 2012, ISSN 1541-2016 pages 1059 - 1067, XP055342304
- YIP, C. ET AL.: 'Primary esophageal cancer: heterogeneity as potential prognostic biomarker in patients treated with definitive chemotherapy and radiation therapy.' RADIOLOGY vol. 270, no. 1, January 2014, ISSN 0033-8419 pages 141 - 148, XP055342306

### SEQUENCE LISTING

<110> Keio University
<120> Heterogeneity marker of cancer and use thereof
<130> PC-22151
<150> JP2015-133033
   <151> 2015-07-01
<160> 34
<170> PatentIn version 3.5
<210> 1
   <211> 20
   <212> DNA
   <213> Artificial Sequence
<220>
   <223> primer ZEB1 Fw
<400> 1
   ggcagagaat gagggagaag 20
<210> 2
   <211> 23
   <212> DNA
   <213> Artificial Sequence
<220>
   <223> primer ZEB1 Rv
<400> 2
   cttcagacac ttgctcacta ctc 23
<210> 3
   <211> 17
   <212> DNA
   <213> Artificial Sequence
<220>
   <223> primer CLDN1-s
<400> 3
   ctgccttctg ggaggtg 17
<210> 4
   <211> 16
   <212> DNA
   <213> Artificial Sequence
<220>
   <223> primer CLDN1-as
<400> 4
   cgctggaagg tgcagg 16
<210> 5
   <211> 21
   <212> DNA
   <213> Artificial Sequence
<220>
   <223> primer CALML3-F
<400> 5
   ggagaagctg agtgacgagg a 21
<210> 6
   <211> 20
   <212> DNA
   <213> Artificial Sequence
<220>
   <223> primer CALML3-R
<400> 6
   acaccagcac acggacaaac 20
<210> 7
   <211> 18
   <212> DNA
   <213> Artificial Sequence
<220>
   <223> primer BGN-F
<400> 7
   atccacgaca accgcatc 18
<210> 8
   <211> 21
   <212> DNA
   <213> Artificial Sequence
<220>
   <223> primer BGN-R
<400> 8
   ccaggttcaa agccactgtt c 21
<210> 9
   <211> 19
   <212> DNA
   <213> Artificial Sequence
<220>
   <223> primer CLCA2-F
<400> 9
   cccagcccac tctattcca 19
<210> 10
   <211> 22
   <212> DNA
   <213> Artificial Sequence
<220>
   <223> primer CLCA2-R
<400> 10
   gctcctcctc atttctgcct ac 22
<210> 11
   <211> 18
   <212> DNA
   <213> Artificial Sequence
<220>
   <223> primer CSTA-F
<400> 11
   cgccactcca gaaatcca 18
<210> 12
   <211> 21
   <212> DNA
   <213> Artificial Sequence
<220>
   <223> primer CSTA-R
<400> 12
   ctgcacagct tccaattttc c 21
<210> 13
   <211> 23
   <212> DNA
   <213> Artificial Sequence
<220>
   <223> primer ALDH1A1-F
<400> 13
   gccataacaa tctcctctgc tct 23
<210> 14
   <211> 21
   <212> DNA
   <213> Artificial Sequence
<220>
   <223> primer ALDH1A1-R
<400> 14
   cccagttctc ttccatttcc a 21
<210> 15
   <211> 22
   <212> DNA
   <213> Artificial Sequence
<220>
   <223> primer NGFR-F
<400> 15
   tccctgtcta ttgctccatc ct 22
<210> 16
   <211> 19
   <212> DNA
   <213> Artificial Sequence
<220>
   <223> primer NGFR-R
<400> 16
   gctgttggct ccttgcttg 19
<210> 17
   <211> 25
   <212> DNA
   <213> Artificial Sequence
<220>
   <223> primer S100A8-F
<400> 17
   gagaccgagt gtcctcagta tatca 25
<210> 18
   <211> 20
   <212> DNA
   <213> Artificial Sequence
<220>
   <223> primer S100A8-R
<400> 18
   cacgcccatc tttatcacca 20
<210> 19
   <211> 21
   <212> DNA
   <213> Artificial Sequence
<220>
   <223> primer ELN-F
<400> 19
   gcagctaaat acggtgctgc t 21
<210> 20
   <211> 20
   <212> DNA
   <213> Artificial Sequence
<220>
   <223> primer ELN-R
<400> 20
   cctgggaaaa tgggagacaa 20
<210> 21
   <211> 21
   <212> DNA
   <213> Artificial Sequence
<220>
   <223> primer SNURF-F
<400> 21
   atcctgcaag atggccgaat c 21
<210> 22
   <211> 22
   <212> DNA
   <213> Artificial Sequence
<220>
   <223> primer SNURF-R
<400> 22
   tggttgcttc gcattctttg gc 22
<210> 23
   <211> 22
   <212> DNA
   <213> Artificial Sequence
<220>
   <223> primer LGALS7/7B-F
<400> 23
   tgccagcagg ttccatgtaa ac 22
<210> 24
   <211> 22
   <212> DNA
   <213> Artificial Sequence
<220>
   <223> primer LGALS7/7B-R
<400> 24
   ttgctccttg ctgttgaaga cc 22
<210> 25
   <211> 1417
   <212> DNA
   <213> Homo sapiens
<220>
   <221> misc_feature
   <223> CALML3
<400> 25
   agggcagggg cgtctgccaa tgatggggga ggactctgct gcttcttaag ctccagcgtc 60
   tcaagccagg gcgagacagc ccgccggccg cccggatctc cacctgccac cccagagctg      120
   ggacagcagc cgggctgcgg cactgggagg gagaccccac agtggcctct tctgccaccc      180
   acgcccccac ccctggcatg gccgaccagc tgactgagga gcaggtcaca gaattcaagg      240
   aggccttctc cctgtttgac aaggatgggg acggctgcat caccacccgc gagctgggca      300
   cggtcatgcg gtccctgggc cagaacccca cggaggccga gctgcgggac atgatgagtg      360
   agatcgaccg ggacggcaac ggcaccgtgg acttccccga gttcctgggc atgatggcca      420
   ggaagatgaa ggacacggac aacgaggagg agatccgcga ggccttccgc gtgttcgaca      480
   aggacggcaa cggcttcgtc agcgccgccg agctgcgaca cgtcatgacc cggctggggg      540
   agaagctgag tgacgaggag gtggacgaga tgatccgggc cgcggacacg gacggagacg      600
   gacaggtgaa ctacgaggag tttgtccgtg tgctggtgtc caagtgaggc cggcgcccac      660
   catgctcctg ggcgcccacg cggcccacag ggcaagaacc cggggcctcc cgcctcctcc      720
   cccatccccc tgcctcccct gggcactgtg gcttcctcct gcgcctggtt gattcagccc      780
   acctctctgc atcccgcttc ccgcgtctct tctctgcact cctgccgacc ttcccacctg      840
   ctcgtctgaa tgacacggaa cgctcccact gcaggcaaac cgtgacgccc tccccactcg      900
   ggagaagcag agctgacctt aggaccgagc accagggcag gttgcgctga ctctgcggcc      960
   ctccaggacg gacaccgggt gaccccttag ggcacccagg caagatccct aagaggcacc     1020
   caatgcccag gccagggggg ctgcagccct cagcccccgc caggattccc gcaggctcct     1080
   ggactggaag ctccctccgc ggtcggattc tggagggtgg gaggcatctt ggcctgcagt     1140
   aagcggtgct gacggggact ctggccacag aggtcaggcc tcctgaaaac agcactgcct     1200
   tccgcgctgc cccagcttgc cccattcctt gtccgccaac ccaccgtgat tcatcttctg     1260
   aagctgggag tgaaactggg tcagctgtaa cctgttccta ttcatctgga aggagggagg     1320
   cttggatgag caggggatga gagctgcagg gaaataaatg agatattcgt ccttatttca     1380
   aaaaaaaaaa aaaaaaaaaa aaaaaaaaaa aaaaaaa                              1417
<210> 26
   <211> 2470
   <212> DNA
   <213> Homo sapiens
<220>
   <221> misc_feature
   <223> BGN
<400> 26
   cctttcctcc ctccccgccc tctccccgct gtcccctccc cgtcggcccg cctgcccagc 60
   ctttagcctc ccgcccgccg cctctgtctc cctctctcca caaactgccc aggagtgagt 120
   agctgctttc ggtccgccgg acacaccgga cagatagacg tgcggacggc ccaccacccc 180
   agcccgccaa ctagtcagcc tgcgcctggc gcctcccctc tccaggtcca tccgccatgt 240
   ggcccctgtg gcgcctcgtg tctctgctgg ccctgagcca ggccctgccc tttgagcaga 300
   gaggcttctg ggacttcacc ctggacgatg ggccattcat gatgaacgat gaggaagctt 360
   cgggcgctga cacctcgggc gtcctggacc cggactctgt cacacccacc tacagcgcca 420
   tgtgtccttt cggctgccac tgccacctgc gggtggttca gtgctccgac ctgggtctga 480
   agtctgtgcc caaagagatc tcccctgaca ccacgctgct ggacctgcag aacaacgaca 540
   tctccgagct ccgcaaggat gacttcaagg gtctccagca cctctacgcc ctcgtcctgg 600
   tgaacaacaa gatctccaag atccatgaga aggccttcag cccactgcgg aagctgcaga 660
   agctctacat ctccaagaac cacctggtgg agatcccgcc caacctaccc agctccctgg 720
   tggagctccg catccacgac aaccgcatcc gcaaggtgcc caagggagtg ttcagcgggc 780
   tccggaacat gaactgcatc gagatgggcg ggaacccact ggagaacagt ggctttgaac 840
   ctggagcctt cgatggcctg aagctcaact acctgcgcat ctcagaggcc aagctgactg 900
   gcatccccaa agacctccct gagaccctga atgaactcca cctagaccac aacaaaatcc 960
   aggccatcga actggaggac ctgcttcgct actccaagct gtacaggctg ggcctaggcc 1020
   acaaccagat caggatgatc gagaacggga gcctgagctt cctgcccacc ctccgggagc 1080
   tccacttgga caacaacaag ttggccaggg tgccctcagg gctcccagac ctcaagctcc 1140
   tccaggtggt ctatctgcac tccaacaaca tcaccaaagt gggtgtcaac gacttctgtc 1200
   ccatgggctt cggggtgaag cgggcctact acaacggcat cagcctcttc aacaaccccg 1260
   tgccctactg ggaggtgcag ccggccactt tccgctgcgt cactgaccgc ctggccatcc 1320
   agtttggcaa ctacaaaaag tagaggcagc tgcagccacc gcggggcctc agtgggggtc 1380
   tctggggaac acagccagac atcctgatgg ggaggcagag ccaggaagct aagccagggc 1440
   ccagctgcgt ccaacccagc cccccacctc gggtccctga ccccagctcg atgccccatc 1500
   accgcctctc cctggctccc aagggtgcag gtgggcgcaa ggcccggccc ccatcacatg 1560
   ttcccttggc ctcagagctg cccctgctct cccaccacag ccacccagag gcaccccatg 1620
   aagctttttt ctcgttcact cccaaaccca agtgtccaag gctccagtcc taggagaaca 1680
   gtccctgggt cagcagccag gaggcggtcc ataagaatgg ggacagtggg ctctgccagg 1740
   gctgccgcac ctgtccagac acacatgttc tgttcctcct cctcatgcat ttccagcctt 1800
   tcaaccctcc ccgactctgc ggctcccctc agcccccttg caagttcatg gcctgtccct 1860
   cccagacccc tgctccactg gcccttcgac cagtcctccc ttctgttctc tctttccccg 1920
   tccttcctct ctctctctct ctctctctct ctctctttct gtgtgtgtgt gtgtgtgtgt 1980
   gtgtgtgtgt gtgtgtgtgt gtgtcttgtg cttcctcaga cctttctcgc ttctgagctt 2040
   ggtggcctgt tccctccatc tctccgaacc tggcttcgcc tgtccctttc actccacacc      2100
   ctctggcctt ctgccttgag ctgggactgc tttctgtctg tccggcctgc acccagcccc      2160
   tgcccacaaa accccaggga cagcagtctc cccagcctgc cctgctcagg ccttgccccc      2220
   aaacctgtac tgtcccggag gaggttggga ggtggaggcc cagcatcccg cgcagatgac      2280
   accatcaacc gccagagtcc cagacaccgg ttttcctaga agcccctcac ccccactggc      2340
   ccactggtgg ctaggtctcc ccttatcctt ctggtccagc gcaaggaggg gctgcttctg      2400
   aggtcggtgg ctgtctttcc attaaagaaa caccgtgcaa cgtggctccg tgaaaaaaaa      2460
   aaaaaaaaaa                                                             2470
<210> 27
   <211> 4043
   <212> DNA
   <213> Homo sapiens
<220>
   <221> misc_feature
   <223> CLCA2
<400> 27
   tcccagatgg atccacccca gacttttcaa agaagacacc tccttcatct tgtgttctaa      60
   aaccttgcaa gttcaggaag aaaccatctg catccatatt gaaaacctga cacaatgtat     120
   gcagcaggct cagtgtgagt gaactggagg cttctctaca acatgaccca aaggagcatt     180
   gcaggtccta tttgcaacct gaagtttgtg actctcctgg ttgccttaag ttcagaactc     240
   ccattcctgg gagctggagt acagcttcaa gacaatgggt ataatggatt gctcattgca     300
   attaatcctc aggtacctga gaatcagaac ctcatctcaa acattaagga aatgataact     360
   gaagcttcat tttacctatt taatgctacc aagagaagag tatttttcag aaatataaag     420
   attttaatac ctgccacatg gaaagctaat aataacagca aaataaaaca agaatcatat     480
   gaaaaggcaa atgtcatagt gactgactgg tatggggcac atggagatga tccatacacc     540
   ctacaataca gagggtgtgg aaaagaggga aaatacattc atttcacacc taatttccta     600
   ctgaatgata acttaacagc tggctacgga tcacgaggcc gagtgtttgt ccatgaatgg     660
   gcccacctcc gttggggtgt gttcgatgag tataacaatg acaaaccttt ctacataaat     720
   gggcaaaatc aaattaaagt gacaaggtgt tcatctgaca tcacaggcat ttttgtgtgt     780
   gaaaaaggtc cttgccccca agaaaactgt attattagta agctttttaa agaaggatgc     840
   acctttatct acaatagcac ccaaaatgca actgcatcaa taatgttcat gcaaagttta     900
   tcttctgtgg ttgaattttg taatgcaagt acccacaacc aagaagcacc aaacctacag     960
   aaccagatgt gcagcctcag aagtgcatgg gatgtaatca cagactctgc tgactttcac    1020
   cacagctttc ccatgaatgg gactgagctt ccacctcctc ccacattctc gcttgtacag 1080
   gctggtgaca aagtggtctg tttagtgctg gatgtgtcca gcaagatggc agaggctgac 1140
   agactccttc aactacaaca agccgcagaa ttttatttga tgcagattgt tgaaattcat 1200
   accttcgtgg gcattgccag tttcgacagc aaaggagaga tcagagccca gctacaccaa 1260
   attaacagca atgatgatcg aaagttgctg gtttcatatc tgcccaccac tgtatcagct 1320
   aaaacagaca tcagcatttg ttcagggctt aagaaaggat ttgaggtggt tgaaaaactg 1380
   aatggaaaag cttatggctc tgtgatgata ttagtgacca gcggagatga taagcttctt 1440
   ggcaattgct tacccactgt gctcagcagt ggttcaacaa ttcactccat tgccctgggt 1500
   tcatctgcag ccccaaatct ggaggaatta tcacgtctta caggaggttt aaagttcttt 1560
   gttccagata tatcaaactc caatagcatg attgatgctt tcagtagaat ttcctctgga 1620
   actggagaca ttttccagca acatattcag cttgaaagta caggtgaaaa tgtcaaacct 1680
   caccatcaat tgaaaaacac agtgactgtg gataatactg tgggcaacga cactatgttt 1740
   ctagttacgt ggcaggccag tggtcctcct gagattatat tatttgatcc tgatggacga 1800
   aaatactaca caaataattt tatcaccaat ctaacttttc ggacagctag tctttggatt 1860
   ccaggaacag ctaagcctgg gcactggact tacaccctga acaataccca tcattctctg 1920
   caagccctga aagtgacagt gacctctcgc gcctccaact cagctgtgcc cccagccact 1980
   gtggaagcct ttgtggaaag agacagcctc cattttcctc atcctgtgat gatttatgcc 2040
   aatgtgaaac agggatttta tcccattctt aatgccactg tcactgccac agttgagcca 2100
   gagactggag atcctgttac gctgagactc cttgatgatg gagcaggtgc tgatgttata 2160
   aaaaatgatg gaatttactc gaggtatttt ttctcctttg ctgcaaatgg tagatatagc 2220
   ttgaaagtgc atgtcaatca ctctcccagc ataagcaccc cagcccactc tattccaggg 2280
   agtcatgcta tgtatgtacc aggttacaca gcaaacggta atattcagat gaatgctcca 2340
   aggaaatcag taggcagaaa tgaggaggag cgaaagtggg gctttagccg agtcagctca 2400
   ggaggctcct tttcagtgct gggagttcca gctggccccc accctgatgt gtttccacca 2460
   tgcaaaatta ttgacctgga agctgtaaaa gtagaagagg aattgaccct atcttggaca 2520
   gcacctggag aagactttga tcagggccag gctacaagct atgaaataag aatgagtaaa 2580
   agtctacaga atatccaaga tgactttaac aatgctattt tagtaaatac atcaaagcga 2640
   aatcctcagc aagctggcat cagggagata tttacgttct caccccaaat ttccacgaat 2700
   ggacctgaac atcagccaaa tggagaaaca catgaaagcc acagaattta tgttgcaata 2760
   cgagcaatgg ataggaactc cttacagtct gctgtatcta acattgccca ggcgcctctg 2820
   tttattcccc ccaattctga tcctgtacct gccagagatt atcttatatt gaaaggagtt 2880
   ttaacagcaa tgggtttgat aggaatcatt tgccttatta tagttgtgac acatcatact 2940
   ttaagcagga aaaagagagc agacaagaaa gagaatggaa caaaattatt ataaataaat 3000
   atccaaagtg tcttccttct tagatataag acccatggcc ttcgactaca aaaacatact 3060
   aacaaagtca aattaacatc aaaactgtat taaaatgcat tgagtttttg tacaatacag 3120
   ataagatttt tacatggtag atcaacaaat tctttttggg ggtagattag aaaaccctta 3180
   cactttggct atgaacaaat aataaaaatt attctttaaa gtaatgtctt taaaggcaaa 3240
   gggaagggta aagtcggacc agtgtcaagg aaagtttgtt ttattgaggt ggaaaaatag 3300
   ccccaagcag agaaaaggag ggtaggtctg cattataact gtctgtgtga agcaatcatt 3360
   tagttacttt gattaatttt tcttttctcc ttatctgtgc agaacaggtt gcttgtttac 3420
   aactgaagat catgctatat tttatatatg aagcccctaa tgcaaagctc tttacctctt 3480
   gctattttgt tatatatatt acagatgaaa tctcactgct aatgctcaga gatctttttt 3540
   cactgtaaga ggtaaccttt aacaatatgg gtattacctt tgtctcttca taccggtttt 3600
   atgacaaagg tctattgaat ttatttgttt gtaagtttct actcccatca aagcagcttt 3660
   ctaagttatt gccttggtta ttatggatga tagttatagc ccttataatg ccttaactaa 3720
   ggaagaaaag atgttattct gagtttgttt taatacatat atgaacatat agttttattc 3780
   aattaaacca aagaagaggt cagcagggag atactaacct ttggaaatga ttagctggct 3840
   ctgttttttg gttaaataag agtctttaat cctttctcca tcaagagtta cttaccaagg 3900
   gcaggggaag ggggatatag aggtcacaag gaaataaaaa tcatctttca tctttaattt 3960
   tactccttcc tcttattttt ttaaaagatt atcgaacaat aaaatcattt gcctttttaa 4020
   ttaaaaaaaa aaaaaaaaaa aaa 4043
<210> 28
   <211> 838
   <212> DNA
   <213> Homo sapiens
<220>
   <221> misc_feature
   <223> CSTA
<400> 28
   tgctgtttgt ggaaaataaa gcattctata ggcggagcta gtgaacgcct cttttaaaac 60
   acgagtctcc acacttccct gttcactttg gttccagcat cctgtccagc aaagaagcaa 120
   tcagccaaaa tgatacctgg aggcttatct gaggccaaac ccgccactcc agaaatccag 180
   gagattgttg ataaggttaa accacagctt gaagaaaaaa caaatgagac ttacggaaaa 240
   ttggaagctg tgcagtataa aactcaagtt gttgctggaa caaattacta cattaaggta 300
   cgagcaggtg ataataaata tatgcacttg aaagtattca aaagtcttcc cggacaaaat 360
   gaggacttgg tacttactgg ataccaggtt gacaaaaaca aggatgacga gctgacgggc 420
   ttttagcagc atgtacccaa agtgttctga ttccttcaac tggctactga gtcatgatcc 480
   ttgctgataa atataaccat caataaagaa gcattctttt ccaaagaaat tatttcttca 540
   attatttctc atttattgta ttaagcagaa attacctttt ctttctcaaa atcagtgtta 600
   ttgctttaga gtataaactc catataaatt gatggcaatt ggaaatctta taaaaactag 660
   tcaagcctaa tgcaactggc taaaggatag taccaccctc acccccacca taggcaggct 720
   ggatcgtgga ctatcaattc accagcctcc ttgttccctg tggctgctga taacccaaca 780
   ttccatctct accctcatac ttcaaaatta aatcaagtat tttacaaaaa aaaaaaaa 838
<210> 29
   <211> 2378
   <212> DNA
   <213> Homo sapiens
<220>
   <221> misc_feature
   <223> ALDH1A1
<400> 29
   gctgcataca cttatcacag gtttcggctt tgtaaattaa ttcatctgca aatagtgcac 60
   tgtctccagg tacaaattcg atgctggagc actggtttct taaggattta agtttaaagt 120
   caaaggcttc ctgccctagg tgttacaaat aagtagtgtc gttttctttt tttgctctga 180
   gtttgttcat ccaatcgtat ccgagtatgc aaataaactt tagcccgtgc agataaaaaa 240
   ggaacaaata aagccaagtg ctctatcaga accaaattgc tgagccagtc acctgtgttc 300
   caggagccga atcagaaatg tcatcctcag gcacgccaga cttacctgtc ctactcaccg 360
   atttgaagat tcaatatact aagatcttca taaacaatga atggcatgat tcagtgagtg 420
   gcaagaaatt tcctgtcttt aatcctgcaa ctgaggagga gctctgccag gtagaagaag 480
   gagataagga ggatgttgac aaggcagtga aggccgcaag acaggctttt cagattggat 540
   ccccgtggcg tactatggat gcttccgaga gggggcgact attatacaag ttggctgatt 600
   taatcgaaag agatcgtctg ctgctggcga caatggagtc aatgaatggt ggaaaactct 660
   attccaatgc atatctgaat gatttagcag gctgcatcaa aacattgcgc tactgtgcag 720
   gttgggctga caagatccag ggccgtacaa taccaattga tggaaatttt tttacatata 780
   caagacatga acctattggt gtatgtggcc aaatcattcc ttggaatttc ccgttggtta 840
   tgctcatttg gaagataggg cctgcactga gctgtggaaa cacagtggtt gtcaaaccag 900
   cagagcaaac tcctctcact gctctccacg tggcatcttt aataaaagag gcagggtttc 960
   ctcctggagt agtgaatatt gttcctggtt atgggcctac agcaggggca gccatttctt 1020
   ctcacatgga tatagacaaa gtagccttca caggatcaac agaggttggc aagttgatca 1080
   aagaagctgc cgggaaaagc aatctgaaga gggtgaccct ggagcttgga ggaaagagcc 1140
   cttgcattgt gttagctgat gccgacttgg acaatgctgt tgaatttgca caccatgggg 1200
   tattctacca ccagggccag tgttgtatag ccgcatccag gatttttgtg gaagaatcaa 1260
   tttatgatga gtttgttcga aggagtgttg agcgggctaa gaagtatatc cttggaaatc 1320
   ctctgacccc aggagtcact caaggccctc agattgacaa ggaacaatat gataaaatac 1380
   ttgacctcat tgagagtggg aagaaagaag gggccaaact ggaatgtgga ggaggcccgt 1440
   gggggaataa aggctacttt gtccagccca cagtgttctc taatgttaca gatgagatgc 1500
   gcattgccaa agaggagatt tttggaccag tgcagcaaat catgaagttt aaatctttag 1560
   atgacgtgat caaaagagca aacaatactt tctatggctt atcagcagga gtgtttacca 1620
   aagacattga taaagccata acaatctcct ctgctctgca ggcaggaaca gtgtgggtga 1680
   attgctatgg cgtggtaagt gcccagtgcc cctttggtgg attcaagatg tctggaaatg 1740
   gaagagaact gggagagtac ggtttccatg aatatacaga ggtcaaaaca gtcacagtga 1800
   aaatctctca gaagaactca taaagaaaat acaagagtgg agagaagctc ttcaatagct 1860
   aagcatctcc ttacagtcac taatatagta gattttaaag acaaaatttt tcttttcttg 1920
   atttttttaa acataagcta aatcatatta gtattaatac tacccataga aaacttgaca 1980
   tgtagcttct tctgaaagaa ttatttgcct tctgaaatgt gacccccaag tcctatccta 2040
   aataaaaaaa gacaaattcg gatgtatgat ctctctagct ttgtcatagt tatgtgattt 2100
   tcctttgtag ctacttttgc aggataataa ttttatagaa aaggaacagt tgcatttagc 2160
   ttctttccct tagtgactct tgaagtactt aacatacacg ttaactgcag agtaaattgc 2220
   tctgttccca gtagttataa agtccttgga ctgttttgaa aagtttccta ggatgtcatg 2280
   tctgcttgtc aaaagaaata atccctgtaa tatttagctg taaactgaat ataaagctta 2340
   ataaaaacaa ccttgcatga ttcttgttaa aaaaaaaa 2378
<210> 30
   <211> 3420
   <212> DNA
   <213> Homo sapiens
<220>
   <221> misc_feature
   <223> NGFR
<400> 30
   agagcgagcc gagccgcggc cagctccggc gggcaggggg ggcgctggag cgcagcgcag 60
   cgcagcccca tcagtccgca aagcggaccg agctggaagt cgagcgctgc cgcgggaggc 120
   gggcgatggg ggcaggtgcc accggccgcg ccatggacgg gccgcgcctg ctgctgttgc 180
   tgcttctggg ggtgtccctt ggaggtgcca aggaggcatg ccccacaggc ctgtacacac 240
   acagcggtga gtgctgcaaa gcctgcaacc tgggcgaggg tgtggcccag ccttgtggag 300
   ccaaccagac cgtgtgtgag ccctgcctgg acagcgtgac gttctccgac gtggtgagcg 360
   cgaccgagcc gtgcaagccg tgcaccgagt gcgtggggct ccagagcatg tcggcgccgt 420
   gcgtggaggc cgacgacgcc gtgtgccgct gcgcctacgg ctactaccag gatgagacga 480
   ctgggcgctg cgaggcgtgc cgcgtgtgcg aggcgggctc gggcctcgtg ttctcctgcc 540
   aggacaagca gaacaccgtg tgcgaggagt gccccgacgg cacgtattcc gacgaggcca 600
   accacgtgga cccgtgcctg ccctgcaccg tgtgcgagga caccgagcgc cagctccgcg 660
   agtgcacacg ctgggccgac gccgagtgcg aggagatccc tggccgttgg attacacggt 720
   ccacaccccc agagggctcg gacagcacag cccccagcac ccaggagcct gaggcacctc 780
   cagaacaaga cctcatagcc agcacggtgg caggtgtggt gaccacagtg atgggcagct 840
   cccagcccgt ggtgacccga ggcaccaccg acaacctcat ccctgtctat tgctccatcc 900
   tggctgctgt ggttgtgggc cttgtggcct acatagcctt caagaggtgg aacagctgca 960
   agcagaacaa gcaaggagcc aacagccggc cagtgaacca gacgccccca ccagagggag 1020
   aaaaactcca cagcgacagt ggcatctccg tggacagcca gagcctgcat gaccagcagc 1080
   cccacacgca gacagcctcg ggccaggccc tcaagggtga cggaggcctc tacagcagcc 1140
   tgcccccagc caagcgggag gaggtggaga agcttctcaa cggctctgcg ggggacacct 1200
   ggcggcacct ggcgggcgag ctgggctacc agcccgagca catagactcc tttacccatg 1260
   aggcctgccc cgttcgcgcc ctgcttgcaa gctgggccac ccaggacagc gccacactgg 1320
   acgccctcct ggccgccctg cgccgcatcc agcgagccga cctcgtggag agtctgtgca 1380
   gtgagtccac tgccacatcc ccggtgtgag cccaaccggg gagcccccgc cccgccccac 1440
   attccgacaa ccgatgctcc agccaacccc tgtggagccc gcacccccac cctttggggg 1500
   gggcccgcct ggcagaactg agctcctctg ggcaggacct cagagtccag gccccaaaac 1560
   cacagccctg tcagtgcagc ccgtgtggcc ccttcacttc tgaccacact tcctgtccag 1620
   agagagaagt gcccctgctg cctccccaac cctgcccctg ccccgtcacc atctcaggcc 1680
   acctgccccc ttctcccaca ctgctaggtg ggccagcccc tcccaccaca gcaggtgtca 1740
   tatatggggg gccaacacca gggatggtac tagggggaag tgacaaggcc ccagagactc 1800
   agagggagga atcgaggaac cagagccatg gactctacac tgtgaacttg gggaacaagg 1860
   gtggcatccc agtggcctca accctccctc agcccctctt gccccccacc ccagcctaag 1920
   atgaagagga tcggaggctt gtcagagctg ggaggggttt tcgaagctca gcccaccccc 1980
   ctcattttgg atataggtca gtgaggccca gggagaggcc atgattcgcc caaagccaga 2040
   cagcaacggg gaggccaagt gcaggctggc accgccttct ctaaatgagg ggcctcaggt 2100
   ttgcctgagg gcgaggggag ggtggcaggt gaccttctgg gaaatggctt gaagccaagt 2160
   cagctttgcc ttccacgctg tctccagacc cccacccctt ccccactgcc tgcccacccg 2220
   tggagatggg atgcttgcct agggcctggt ccatgatgga gtcaggtttg gggttcgtgg 2280
   aaagggtgct gcttccctct gcctgtccct ctcaggcatg cctgtgtgac atcagtggca 2340
   tggctccagt ctgctgccct ccatcccgac atggacccgg agctaacact ggcccctaga 2400
   atcagcctag gggtcaggga ccaaggaccc ctcaccttgc aacacacaga cacacgcaca 2460
   cacacacaca ggaggagaaa tctcactttt ctccatgagt tttttctctt gggctgagac 2520
   tggatactgc ccggggcagc tgccagagaa gcatcggagg gaattgaggt ctgctcggcc 2580
   gtcttcactc gcccccgggt ttggcgggcc aaggactgcc gaccgaggct ggagctggcg 2640
   tctgtcttca agggcttaca cgtggaggaa tgctccccca tcctcccctt ccctgcaaac 2700
   atggggttgg ctgggcccag aaggttgtga tgaagaaaag tgggccagtg tgggaatgcg 2760
   gcaagaagga attgacttcg actgtgacct gtggggattt ctcccagctc tagacaaccc 2820
   tgcaaaggac tgttttttcc tgagcttggc cagaaggggg ccatgaggcc tcagtggact 2880
   ttccaccccc tccctggcct gttctgtttt gcctgaagtt ggagtgagtg tggctcccct 2940
   ctatttagca tgacaagccc caggcaggct gtgcgctgac aaccaccgct ccccagccca 3000
   gggttccccc agccctgtgg aagggactag gagcactgta gtaaatggca attctttgac 3060
   ctcaacctgt gatgagggga ggaaactcac ctgctggccc ctcacctggg cacctgggga 3120
   gtgggacaga gtctgggtgt atttattttc ctccccagca ggtggggagg gggtttgggg 3180
   gcttgcaagt atgttttagc atgtgtttgg ttctggggcc cctttttact ccccttgagc 3240
   tgagatggaa cccttttggc ccccgagctg ggggccatga gctccagacc cccagcaacc 3300
   ctcctatcac ctcccctcct tgcctcctgt gtaatcattt cttgggccct cctgaaactt 3360
   acacacaaaa cgttaagtga tgaacattaa atagcaaaga aagaaaaata gtacaaagag 3420
<210> 31
   <211> 532
   <212> DNA
   <213> Homo sapiens
<220>
   <221> misc_feature
   <223> S100A8
<400> 31
   gagaaaccag agactgtagc aactctggca gggagaagct gtctctgatg gcctgaagct 60
   gtgggcagct ggccaagcct aaccgctata aaaaggagct gcctctcagc cctgcatgtc 120
   tcttgtcagc tgtctttcag aagacctggt ggggcaagtc cgtgggcatc atgttgaccg 180
   agctggagaa agccttgaac tctatcatcg acgtctacca caagtactcc ctgataaagg      240
   ggaatttcca tgccgtctac agggatgacc tgaagaaatt gctagagacc gagtgtcctc      300
   agtatatcag gaaaaagggt gcagacgtct ggttcaaaga gttggatatc aacactgatg      360
   gtgcagttaa cttccaggag ttcctcattc tggtgataaa gatgggcgtg gcagcccaca      420
   aaaaaagcca tgaagaaagc cacaaagagt agctgagtta ctgggcccag aggctgggcc      480
   cctggacatg tacctgcaga ataataaagt catcaatacc tcaaaaaaaa aa              532
<210> 32
   <211> 3789
   <212> DNA
   <213> Homo sapiens
<220>
   <221> misc_feature
   <223> ELN
<400> 32
<210> 33
   <211> 1575
   <212> DNA
   <213> Homo sapiens
<220>
   <221> misc_feature
   <223> SNURF
<400> 33
   aaacaagcac gcctgcgcgg ccgcagaggc aggctggcgc gcatgctcag gcggggatgt 60
   gtgcgaagcc tgccgctgct gcagcgagtc tggcgcagag tggagcggcc gccggagatg 120
   cctgacgcat ctgtctgagg agcggtcagt gacgcgatgg agcgggcaag ggatcgctta 180
   cacctgagac gaactacaga acagcacgta ccagaggtgg aagtccaagt caaacgcaga 240
   aggactgcct cactgagcaa ccaagagtgt cagttgtacc cgaggcgttc tcagcagcag 300
   caagtacctg tggtggattt ccaggctgaa ctgaggcagg cattcttagc tgagacacca 360
   agaggtggtt aaagccatat tggagtagcg aggaatctga ttccaagcaa aaaccaggct 420
   ccatctactc tttgaagctt ctgcccagct tgcattgttt ctaggagaac ctgcgtcata 480
   cctttatcta tagccttccc ctaggtcttc agaagcatca agttttaact gtggacattg 540
   gatttggtgg aacagcaatc atgactgttg gcaagagtag caagatgctg cagcacattg 600
   actatagaat gagatgtatc ctgcaagatg gccgaatctt cattggcacc tttaaggctt 660
   ttgacaagca tatgaatttg atcctctgtg attgtgatga gttcagaaag atcaagccaa 720
   agaatgcgaa gcaaccagag cgtgaagaaa agcgggtttt gggtctggtg ttgctgcgtg 780
   gggagaactt ggtatccatg actgtggagg ggccaccccc caaagatact ggcattgctc 840
   gggtaccact tgctggagct gctggaggcc ctggggttgg tagggcagct ggtagaggag 900
   taccagctgg tgtgccaatt ccccaggccc ctgctggatt ggcaggccct gtccgaggag 960
   ttgggggacc atcccagcag gtaatgactc cacagggaag aggcactgta gcagctgctg 1020
   ctgttgctgc gactgccagt attgctggag ccccaacaca gtacccacca ggacggggca 1080
   ctccgccccc acccgtcggc agagcaaccc cacctccagg cattatggct cctccacctg 1140
   gtatgagacc acccatgggc ccaccaattg ggcttccccc tgctcgaggg acgccaatag 1200
   gcatgccgcc tccgggaatg agaccccctc caccaggcat tagaggtcca cctcccccag 1260
   gaatgcgtcc accaagacct tagcatactg ttgatccatc tcagtcactt tttcccctgc 1320
   aatgcgtctt gtgaaattgt gtagagtgtt tgtgagcttt ttgttccctc attctgcatt 1380
   aataatagct aataataaat gcatagagca attaaactgt gaggtactgt tgtatatatt 1440
   tttttgcctg ttgattttga tgagatctta agttactgtg gatgagggtg atgcctatta 1500
   agcagttgat tcaaatcata ttctctttaa ttcttaggat aaaaaggttt tctgctatct 1560
   aaaaaaaaaa aaaaa 1575
<210> 34
   <211> 515
   <212> DNA
   <213> Homo sapiens
<220>
   <221> misc_feature
   <223> LGALS7
<400> 34
   acggctgccc aacccggtcc cagccatgtc caacgtcccc cacaagtcct cactgcccga      60
   gggcatccgc cctggcacgg tgctgagaat tcgcggcttg gttcctccca atgccagcag     120
   gttccatgta aacctgctgt gcggggagga gcagggctcc gatgccgcgc tgcatttcaa     180
   cccccggctg gacacgtcgg aggtggtctt caacagcaag gagcaaggct cctggggccg     240
   cgaggagcgc gggccgggcg ttcctttcca gcgcgggcag cccttcgagg tgctcatcat     300
   cgcgtcagac gacggcttca aggccgtggt tggggacgcc cagtaccacc acttccgcca     360
   ccgcctgccg ctggcgcgcg tgcgcctggt ggaggtgggc ggggacgtgc agctggactc     420
   cgtgaggatc ttctgagcag aagcccaggc gggcccgggg ccttggctgg caaataaagc     480
   gttagcccgc agcgaaaaaa aaaaaaaaaa aaaaa                                515

## Description

### Technical Field

The present invention relates to a use of a genetic marker for determining whether or not a cancer tissue contains a heterogeneous cancer cell population, a use of a protein marker for determining whether or not a cancer tissue contains a heterogeneous cancer cell population, a use of a kit for determining whether or not a cancer tissue contains a heterogeneous cancer cell population, and a method for determining whether or not a cancer tissue sample contains a heterogeneous cancer cell population. Priority is claimed on Japanese Patent Application No. 2015-133033, filed on July 1, 2015.

### Background Art

Cancer cells having different properties are known to be mixed in one cancer tissue. Such a state is called intratumor heterogeneity and is thought to be one factor that contributes to making cancer treatment difficult (see, for example, NPL 1, NPL 2, and NPL 3).

Methods for diagnosing of certain cancer types by detecting expression levels of certain markers (in particular CALM3 for lung cancer and ALDH1 for breast cancer) are described in the prior art (see, for example, PL 1 and PL 2).

### Citation List

### Non-Patent Literature

NPL 1: Inda M. M., et al., Tumor heterogeneity is an active process maintained by a mutant EGFR-induced cytokine circuit in glioblastoma., Genes Dev., 24 (16), pp 1731-1745, 2010;
NPL 2: Navin N., et al., Inferring tumor progression from genomic heterogeneity, Genome Res., 20(1), pp 68-80, 2010;
NPL 3: Martelotto L. G, et al., Breast cancer intra-tumor heterogeneity, Breast Cancer Res., 16:R48, 2014.

### Patent Literature

PL 1: WO 2006/053442
PL 2: CN 101993944

### Summary of Invention

### Technical Problem

Conventionally, there has not been known a method for determining heterogeneity of a cancer tissue (a state in which the cancer tissue contains a heterogeneous cancer cell population). Therefore, it is an object of the present invention to provide a genetic marker for determining whether or not a cancer tissue contains a heterogeneous cancer cell population. It is another object of the present invention to provide a protein marker for determining whether or not a cancer tissue contains a heterogeneous cancer cell population, a kit for determining whether or not a cancer tissue contains a heterogeneous cancer cell population, and a method for determining whether or not a cancer tissue contains a heterogeneous cancer cell population.

### Solution to Problem

The present invention is as follows.
(1) Use of a genetic marker for determining whether or not a cancer tissue contains a heterogeneous cancer cell population, the genetic marker being selected from the group consisting of Calmodulin-like protein 3 (CALML3), Biglycan (BGN), Chloride channel accessory 2 (CLCA2), Cystatin A (CSTA), Aldehyde dehydrogenase 1 family, member A1 (ALDH1A1), Nerve growth factor receptor (NGFR), S100-calcium-binding protein A8 (S100A8), Elastin (ELN), SNRPN upstream reading frame (SNURF), and Galectin-7 (LGALS7).
(2) Use of a protein marker for determining whether or not a cancer tissue contains a heterogeneous cancer cell population, the protein marker being encoded by a gene selected from the group consisting of CALML3, BGN, CLCA2, CSTA, ALDH1A1, NGFR, S100A8, ELN, SNURF, and LGALS7.
(3) Use of a gene selected from the group consisting of CALML3, BGN, CLCA2, CSTA, NGFR, S100A8, ELN, SNURF, and LGALS7, or a protein encoded by the gene, for detecting a heterogeneous cancer cell population to predict a prognosis of a cancer patient.
(4) Use of a kit for determining whether or not a cancer tissue contains a heterogeneous cancer cell population, the kit comprising a primer set for amplifying cDNA of at least one gene selected from the group consisting of CALML3, BGN, CLCA2, CSTA, ALDH1A1, NGFR, S100A8, ELN, SNURF, and LGALS7; a probe that specifically hybridizes to mRNA of at least one gene selected from the group consisting of CALML3, BGN, CLCA2, CSTA, ALDH1A1, NGFR, S100A8, ELN, SNURF, and LGALS7; or a specific binding substance to a protein encoded by at least one gene selected from the group consisting of CALML3, BGN, CLCA2, CSTA, ALDH1A1, NGFR, S100A8, ELN, SNURF, and LGALS7.
(5) Use of a kit for detecting a heterogeneous cancer cell population to predict a prognosis of a cancer patient, the kit comprising a primer set for amplifying cDNA of at least one gene selected from the group consisting of CALML3, BGN, CLCA2, CSTA, NGFR, S100A8, ELN, SNURF, and LGALS7; a probe that specifically hybridizes to mRNA of at least one gene selected from the group consisting of CALML3, BGN, CLCA2, CSTA, NGFR, S100A8, ELN, SNURF, and LGALS7; or a specific binding substance to a protein encoded by at least one gene selected from the group consisting of CALML3, BGN, CLCA2, CSTA, NGFR, S100A8, ELN, SNURF, and LGALS7.
(6) A method for determining whether or not a cancer tissue sample contains a heterogeneous cancer cell population, including a detection step of detecting the expression of at least one gene selected from the group consisting of CALML3, BGN, CLCA2, CSTA, ALDH1A1, NGFR, S100A8, ELN, SNURF, and LGALS7 in a cancer tissue sample; and a step of determining that the cancer tissue sample contains a heterogeneous cancer cell population in the case where the expression of the gene is detected.
(7) The determination method according to (6), (i) in which the detection step is carried out by detecting mRNA of the gene; or (ii) the detection step is carried out by detecting a protein encoded by the gene.
(8) The determination method according to (6) or (7), in which the cancer tissue sample is derived from breast cancer, melanoma, lung cancer, or pancreatic cancer.
(9) The determination method according to (8), in which the breast cancer is an estrogen receptor(-) progesterone receptor(-) HER2(-) breast cancer.
(10) The determination method according to any one of (6) to (9), in which the heterogeneous cancer cell population includes ZEB1(+) CLDN1(-) cells and ZEB1(-) CLDN1(+) cells.
(11) A method for detecting a heterogeneous cancer cell population to predict a prognosis of cancer, including a detection step of detecting the expression of at least one gene selected from the group consisting of CALML3, BGN, CLCA2, CSTA, NGFR, S100A8, ELN, SNURF, and LGALS7 in a cancer tissue sample; and a step of determining that a patient from whom the cancer tissue sample is derived has a poor prognosis in the case where the expression of the gene is detected.
(12) A method for preparing a heterogeneous cancer cell population expressing a gene selected from the group consisting of CALML3, BGN, CLCA2, CSTA, ALDH1A1, NGFR, S100A8, ELN, SNURF, and LGALS7, or a protein encoded by the gene, comprising the steps of mixing a cell line that does not form a tumor upon single transplantation with a cell line that forms a tumor even upon single transplantation and transplanting such a cell line mixture into a nude mouse.
(13) The method for preparing a heterogeneous cancer cell population according to (12), wherein the heterogeneous cancer cell population is isolated from the nude mouse.

### Advantageous Effects of Invention

According to the present invention, it is possible to determine whether or not a cancer tissue contains a heterogeneous cancer cell population by using the genetic marker, protein marker, gene, kits, or methods specified in the claims.

### Brief Description of Drawings

FIG. 1(a) is a graph showing the analysis results of ZEB1 gene expression in a plurality of breast cancer cell lines. FIG. 1(b) is a graph showing the analysis results of CLDN1 gene expression in a plurality of breast cancer cell lines.
FIG. 2 is a graph showing the results of Experimental Example 3.
FIG. 3(a) is a photograph showing the results of staining a thin-sectioned tissue sample of a cancer tissue with an anti-ZEB 1 antibody. FIG. 3(b) is a photograph showing the results of staining a thin-sectioned tissue sample having approximately the same field of view as FIG. 3(a) with an anti-CLDNl antibody.
FIGS. 4(a) and 4(b) are graphs showing the results of Experimental Example 6.
FIG. 5 is a graph showing the results of Experimental Example 7.
FIG. 6 is a diagram showing the analysis results of Experimental Example 8.
FIGS. 7(a) to 7(j) are photographs showing the results of Experimental Example 10.
FIGS. 8(a) to 8(j) are graphs showing the results of quantitative real-time PCR in Experimental Example 11.

### Description of Embodiments

### Marker for determining whether or not cancer tissue contains heterogeneous cancer cell population

In one aspect, the present invention provides a use of a genetic marker for determining whether or not a cancer tissue contains a heterogeneous cancer cell population, the genetic marker being selected from the group consisting of Calmodulin-like protein 3 (CALML3, accession number: NM_005185, SEQ ID NO: 25), Biglycan (BGN, accession number: NM_001711, SEQ ID NO: 26), Chloride channel accessory 2 (CLCA2, accession number: NM_006536, SEQ ID NO: 27), Cystatin A (CSTA, (StefinA), accession number: NM_005213, SEQ ID NO: 28), Aldehyde dehydrogenase 1 family, member A1 (ALDH1A1, accession number: NM_000689, SEQ ID NO: 29), Nerve growth factor receptor (NGFR, accession number: NM_002507, SEQ ID NO: 30), S100-calcium-binding protein A8 (S100A8, accession number: NM_002964, SEQ ID NO: 31), Elastin (ELN, accession number: NM_000501, SEQ ID NO: 32), SNRPN upstream reading frame (SNURF, accession number: NM_005678, SEQ ID NO: 33), and Galectin-7 (LGALS7, accession number: NM_002307, SEQ ID NO: 34).

In one embodiment, the marker used may be a cDNA marker for determining whether or not a cancer tissue contains a heterogeneous cancer cell population, which is selected from the group consisting of CALML3 cDNA, BGN cDNA, CLCA2 cDNA, CSTA cDNA, ALDH1A1 cDNA, NGFR cDNA, S100A8 cDNA, ELN cDNA, SNURF cDNA, and LGALS7 cDNA.

As will be described later in the EXAMPLES, the present inventors have produced unexpectedly a cancer tissue composed of heterogeneous cell populations by mixing a cell line that does not form a tumor upon single transplantation with a cell line that forms a tumor even upon single transplantation and transplanting such a cell line mixture into a nude mouse. In addition, the present invention has been completed by identifying CALML3, BGN, CLCA2, CSTA, ALDH1A1, NGFR, S100A8, ELN, SNURF, and LGALS7 as genes which are specifically expressed in a cancer tissue containing a heterogeneous cancer cell population.

Therefore, in the case where the expression of any one or more of these genes is detected in a cancer tissue, it can be determined that the cancer tissue contains a heterogeneous cancer cell population.

In addition, as will be described later in the EXAMPLES, the present inventors have found that a cancer tissue containing a heterogeneous cancer cell population tends to have a poor prognosis. Therefore, a gene selected from the group consisting of CALML3, BGN, CLCA2, CSTA, NGFR, S100A8, ELN, SNURF, and LGALS7, and a cDNA thereof can also be said to be a genetic marker for determining a prognosis of cancer.

In the case where the expression of any one or more of these genes is detected in a cancer tissue, it can be predicted that a cancer patient having such a cancer tissue has a poor prognosis.

In the present specification, the poor prognosis means that cancer cell proliferation is fast, a metastatic ability of cancer is high, a resistance of a cancer tissue to an anticancer drug is high, a survival rate of a patient is low, or the like.

The above-mentioned genetic marker may be a splicing variant of the foregoing gene by alternative splicing or the like. In addition, the above-mentioned genetic marker may be a mutant of the foregoing gene including single nucleotide polymorphism (SNP) and the like.

The marker for determining whether or not a cancer tissue contains a heterogeneous cancer cell population may be a protein encoded by the foregoing gene. That is, the marker for determination may be a protein marker.

Also, like the above-described genetic marker, the protein encoded by a gene selected from the group consisting of CALML3, BGN, CLCA2, CSTA, ALDH1A1, NGFR, S100A8, ELN, SNURF, and LGALS7 can also be said to be a protein marker for determining a prognosis of cancer.

The expression of the foregoing genetic marker or protein marker in a cancer tissue can be detected by RT-PCR, DNA array analysis, Northern blotting, immunostaining, ELISA, Western blotting, flow cytometric analysis, or the like.

Among CALML3, BGN, CLCA2, CSTA, ALDH1A1, NGFR, S100A8, ELN, SNURF, and LGALS7 genes, cDNAs thereof, or proteins encoded by such genes, particularly CALML3, CLCA2, CSTA, and LGALS7 genes, cDNAs thereof, or proteins encoded by such genes are more preferable as the marker for determining whether or not a cancer tissue contains a heterogeneous cancer cell population. As will be described later in the EXAMPLES, the expression of CALML3, CLCA2, CSTA, and LGALS7 genes has actually been confirmed in clinical specimens of human breast cancer.

### Kit for determining whether or not cancer tissue contains heterogeneous cancer cell population

In one aspect, the present invention provides a use of a kit for determining whether or not a cancer tissue contains a heterogeneous cancer cell population, including a primer set for amplifying cDNA of at least one gene selected from the group consisting of CALML3, BGN, CLCA2, CSTA, ALDH1A1, NGFR, S100A8, ELN, SNURF, and LGALS7; a probe that specifically hybridizes to mRNA of at least one gene selected from the group consisting of CALML3, BGN, CLCA2, CSTA, ALDH1A1, NGFR, S100A8, ELN, SNURF, and LGALS7; or a specific binding substance to a protein encoded by at least one gene selected from the group consisting of CALML3, BGN, CLCA2, CSTA, ALDH1A1, NGFR, S100A8, ELN, SNURF, and LGALS7.

As described above, in the case where the expression of any one or more of the foregoing genes is detected in a cancer tissue, it can be predicted that a cancer patient having such a cancer tissue has a poor prognosis. Therefore, the kit used in the present aspect can also be said to be a kit for determining a prognosis of cancer.

### Primer set

The kit used in the present aspect may include a primer set for amplifying cDNA of at least one gene selected from the group consisting of CALML3, BGN, CLCA2, CSTA, ALDH1A1, NGFR, S100A8, ELN, SNURF, and LGALS7.

The sequence of the primer set is not particularly limited as long as it can amplify at least a part of cDNA of these genes. Specific examples of the base sequence of the primer set include those described later in the EXAMPLES.

At least one of the primers constituting the primer set may have a base sequence including an exon-exon boundary in the base sequence of any one of the foregoing genes. Such a primer has a base sequence which does not exist in nature.

### Probe

The kit used in the present aspect may include a probe that specifically hybridizes to mRNA of at least one gene selected from the group consisting of CALML3, BGN, CLCA2, CSTA, ALDH1A1, NGFR, S100A8, ELN, SNURF, and LGALS7.

The probe may be, for example, a nucleic acid fragment having a base sequence complementary to the base sequence of at least a part of the mRNA of the foregoing gene. Further, the probe may have various chemical modifications for the purpose of improving the stability, specificity at the time of hybridization, and the like. For example, a phosphate residue may be substituted with a chemically modified phosphate residue such as phosphorothioate (PS), methylphosphonate, or phosphorodithionate in order to suppress degradation by a hydrolytic enzyme such as nuclease. Also, at least a part of the probe may be constituted of a nucleic acid analog such as peptide nucleic acid (PNA).

In addition, the probe may have a base sequence including an exon-exon boundary in the base sequence of any one of the foregoing genes. Such a probe has a base sequence which does not exist in nature.

The probe may be fixed on a solid phase. Examples of the solid phase include beads, plate-like substrates, membranes, and the like.

For example, the probe may be fixed to the surface of a plate-like substrate to form a microarray. In this case, for example, the expression of the foregoing gene in a cancer tissue can be detected by extracting RNA from a cancer tissue sample, labeling the extracted RNA with a fluorescent substance, hybridizing the labeled extracted RNA with a microarray, and detecting the RNA bound to the probe on the microarray. In the case where the expression of any one or more of the foregoing genes is detected, it can be determined that the cancer tissue contains a heterogeneous cancer cell population.

### Specific binding substance

The kit used in the present aspect may include a specific binding substance to a protein encoded by at least one gene selected from the group consisting of CALML3, BGN, CLCA2, CSTA, ALDH1A1, NGFR, S100A8, ELN, SNURF, and LGALS7.

Examples of the specific binding substance include an antibody, an antibody fragment, and an aptamer. The antibody can be constructed, for example, by immunizing an animal such as a mouse with the foregoing protein as an antigen. Alternatively, the antibody can be constructed by screening an antibody library such as a phage library. Examples of the antibody fragment include Fv, Fab, and scFv. The antibody or antibody fragment may be polyclonal or monoclonal.

The aptamer is a substance having a specific binding ability to a labeling substance. Examples of the aptamer include a nucleic acid aptamer and a peptide aptamer. The nucleic acid aptamer having a specific binding ability to the foregoing protein may be selected by, for example, systematic evolution of ligand by exponential enrichment (SELEX). Further, the peptide aptamer having a specific binding ability to the foregoing protein may be selected by, for example, a two-hybrid method using yeast.

The specific binding substance is not particularly limited as long as it can specifically bind to the foregoing protein, and may be a commercially available product.

For example, the presence of the foregoing protein can be detected by immunostaining the thin-sectioned sample of the fixed cancer tissue using the foregoing specific binding substance. The detection of the presence of the foregoing protein may be carried out by Western blotting, ELISA, flow cytometric analysis, or the like.

The presence of any one or more of the foregoing proteins indicates that a cancer tissue contains a heterogeneous cancer cell population.

### Method for determining whether cancer tissue sample contains heterogeneous cancer cell population

In one aspect, the present invention provides a method for determining whether or not a cancer tissue sample contains a heterogeneous cancer cell population, including a detection step of detecting the expression of at least one gene selected from the group consisting of CALML3, BGN, CLCA2, CSTA, ALDH1A1, NGFR, S100A8, ELN, SNURF, and LGALS7 in a cancer tissue sample; and a step of determining that the cancer tissue sample contains a heterogeneous cancer cell population in the case where the expression of the gene is detected.

As described above, in the case where the expression of any one or more of the foregoing genes is detected in a cancer tissue sample, it can be predicted that a cancer patient having such a cancer tissue has a poor prognosis. Therefore, the determination method of the present embodiment can also be said to be a method for determining a prognosis of cancer.

The above detection step may be carried out by detecting mRNA of the foregoing gene. Alternatively, the detection step may be carried out by detecting a protein encoded by the foregoing gene.

The detection of mRNA of the foregoing gene can be carried out by RT-PCR, DNA array analysis, Northern blotting, or the like. In addition, the detection of the protein encoded by the foregoing gene can be carried out by immunostaining, ELISA, Western blotting, flow cytometric analysis, or the like.

In the case where the expression of any one or more of the foregoing genes is detected, it can be determined that the cancer tissue sample contains a heterogeneous cancer cell population.

The cancer tissue sample may be a sample derived from breast cancer, melanoma, lung cancer, or pancreatic cancer. Some of these cancers contain heterogeneous cancer cell populations, and those with heterogeneous cancer cell populations are known to have a poor prognosis.

The breast cancer may be an estrogen receptor(-) progesterone receptor(-) HER2(-) breast cancer, that is, a breast cancer that does not express an estrogen receptor, a progesterone receptor, and HER2. Such a breast cancer is also referred to as a triple negative breast cancer and it is known to be a breast cancer having a poor prognosis.

In addition, the heterogeneous cancer cell population may be a cell population containing ZEB1(+) CLDN1(-) cells, that is, cells expressing ZEB1 and not expressing CLDN1, and ZEB1(-) CLDN1(+) cells, that is, cells not expressing ZEB1 and expressing CLDN1.

Zinc finger E-box-binding homeobox 1 (ZEB1) is a transcription factor that induces epithelial-mesenchymal transition which is a phenomenon in which an epithelial cell loses its cell polarity or a cell adhesion function to the surrounding cells and obtains a migration and invasion ability so that the epithelial cell changes into a mesenchymal-like cell. Further, Claudin 1 (CLDN1) is a major protein present in a tight junction.

As will be described later in the EXAMPLES, a cancer tissue containing ZEB1(+) CLDN1(-) cells and ZEB1(-) CLDN1(+) cells can be mentioned as an example of the cancer tissue containing a heterogeneous cancer cell population.

### Method for preparing a heterogeneous cancer cell population

In one aspect, the present invention provides a method for preparing a heterogeneous cancer cell population which expresses a gene selected from the group consisting of CALML3, BGN, CLCA2, CSTA, ALDH1A1, NGFR, S100A8, ELN, SNURF, and LGALS7, or a protein encoded by the gene, the method comprising the steps of mixing a cell line that does not form a tumor upon single transplantation with a cell line that forms a tumor even upon single transplantation and transplanting such a cell line mixture into a nude mouse. As will be described later in the EXAMPLES, it is possible to form a heterogeneous cancer cell population as a cancer tissue of a tumor-bearing mouse, for example, by transplanting a mixture of ZEB1(+) CLDN1(-) cancer cells and ZEB1(-) CLDN1(+) cancer cells into an immunodeficient mouse.

### Other disclosure

Also disclosed is a cDNA of a gene selected from the group consisting of CALML3, BGN, CLCA2, CSTA, ALDH1A1, NGFR, S100A8, ELN, SNURF, and LGALS7. As described above, such a cDNA can be used as a genetic marker for determining whether or not a cancer tissue contains a heterogeneous cancer cell population, or as a marker for determining a prognosis of cancer.

Also disclosed is a method for detecting the expression of a gene in a cancer tissue sample, including a step of collecting the cancer tissue sample from a patient; and a step of detecting the expression of a gene selected from the group consisting of CALML3, BGN, CLCA2, CSTA, ALDH1A1, NGFR, S100A8, ELN, SNURF, and LGALS7 or a cDNA thereof in the cancer tissue sample, by polymerase chain reaction (PCR), gene expression analysis using a microarray, or detection of binding with a specific binding substance to a protein encoded by the gene.

Also disclosed is a method for determining whether or not a cancer tissue sample contains a heterogeneous cancer cell population, including a step of collecting the cancer tissue sample from a patient; and a step of detecting the expression of a gene selected from the group consisting of CALML3, BGN, CLCA2, CSTA, ALDH1A1, NGFR, S100A8, ELN, SNURF, and LGALS7 or a cDNA thereof in the cancer tissue sample, by polymerase chain reaction (PCR), gene expression analysis using a microarray, or detection of binding with a specific binding substance to a protein encoded by the gene. The determination method of the present embodiment can also be said to be a method for determining a prognosis of cancer.

### EXAMPLES

Hereinafter, the present invention will be described in more detail with reference to the following Examples, but the present invention is not limited thereto.

### Construction of model mouse having heterogeneous cancer tissue

### Experimental Example 1

### Study on expression of ZEB1 and CLDN1 in breast cancer cell lines

The present inventors have studied the expression of ZEB1 gene and CLDN1 gene in triple negative breast cancer cell lines, MDA-MB-436, BT549, MDA-MB-157, MDA-MB-231, Hs578T, HCC1937, BT20, and MDA-MB-468 cells by quantitative real-time PCR. Primers ZEB1 Fw (SEQ ID NO: 1) and ZEB1 Rv (SEQ ID NO: 2) were used for amplification of the ZEB1 gene. Primers CLDN-s (SEQ ID NO: 3) and CLDN-as (SEQ ID NO: 4) were used for amplification of the CLDN1 gene.

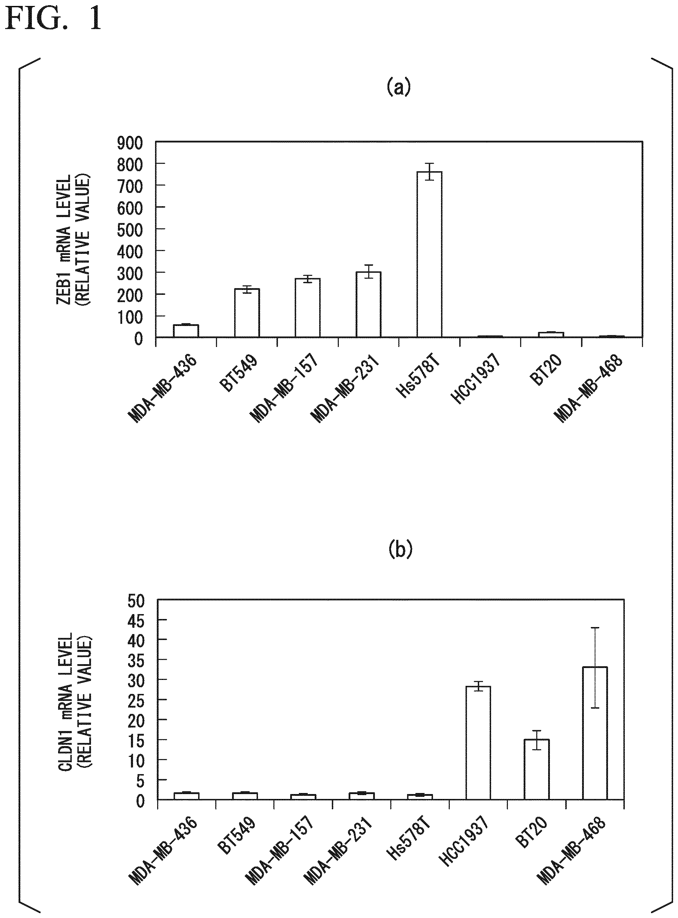

FIG 1(a) is a graph showing the analysis results of the expression of ZEB1 gene in each breast cancer cell line. In addition, FIG. 1(b) is a graph showing the analysis results of the expression of CLDN1 gene in each breast cancer cell line.

As a result, the present inventors have found that the MDA-MB-231 cell line was ZEB1(+) CLDN1(-). The present inventors have also found that the HCC1937 cell line was ZEB1(-) CLDN1(+).

### Experimental Example 2

### Construction of tumor-bearing mouse

The MDA-MB-231 cell line and the HCC1937 cell line were mixed so that the cell number of the HCC1937 cell line was larger than the cell number of the MDA-MB-231 cell line, preferably the cell number of HCC1937 cell line:cell number of MDA-231 cell line was a ratio of 2:1 or more, for example, 9:1. Then, the cell mixture was transplanted into the fourth mammary gland adipose tissue of an immunodeficient mouse (4 weeks old, female, BALB/c-nu) to thereby construct a tumor-bearing mouse (hereinafter, sometimes referred to as "Mix mouse"). Only the MDA-MB-231 cell line and only the HCC1937 cell line were respectively transplanted into the fourth mammary gland adipose tissue of an immunodeficient mouse (4 weeks old, female, BALB/c-nu) to thereby construct tumor-bearing mice (hereinafter, sometimes referred to as "231 mouse" and "1937 mouse", respectively) as controls.

### Experimental Example 3

### Observation of temporal changes in tumor volume

The tumor diameters in the Mix mouse, 231 mouse, and 1937 mouse constructed in Experimental Example 2 were measured over time of about 30 days after the transplantation of the cancer cells, and temporal changes in tumor volume were observed.

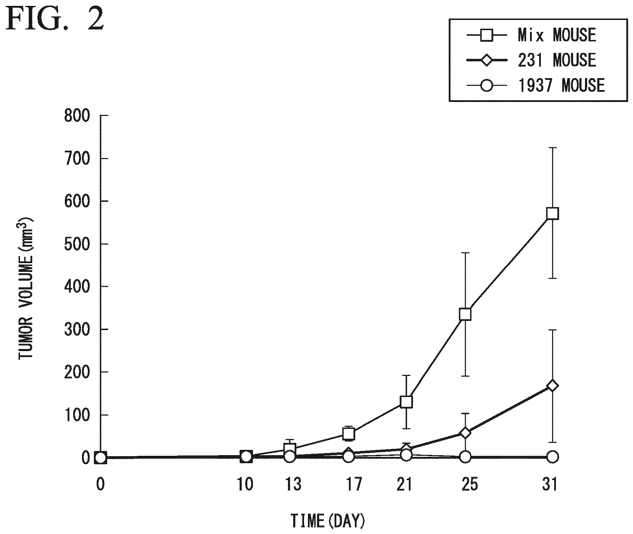

FIG. 2 is a graph showing the temporal changes in tumor volume in each tumor-bearing mouse. As a result, it was found that no tumor was formed in the 1937 mouse into which only the HCC1937 cell line had been transplanted. In addition, a tumor was formed in the Mix mouse into which the MDA-MB-231 cell line had been transplanted in admixture with the HCC1937 cell line, although a tumor was not formed in the 1937 mouse. Further, it was found that a larger tumor was formed in the Mix mouse than the 231 mouse into which only the same number of MDA-MB-231 cell line had been transplanted.

### Experimental Example 4

### Immunostaining of cancer tissue of tumor-bearing mouse

From the Mix mouse constructed in the same manner as in Experimental Example 2, a cancer tissue was excised 16 days after the transplantation of cancer cell lines. Subsequently, the excised cancer tissue was fixed with paraformaldehyde and embedded in paraffin. Subsequently, thin-sectioned tissue samples of the cancer tissue were prepared and the expression of ZEB1 protein and CLDN1 protein was examined by immunostaining.

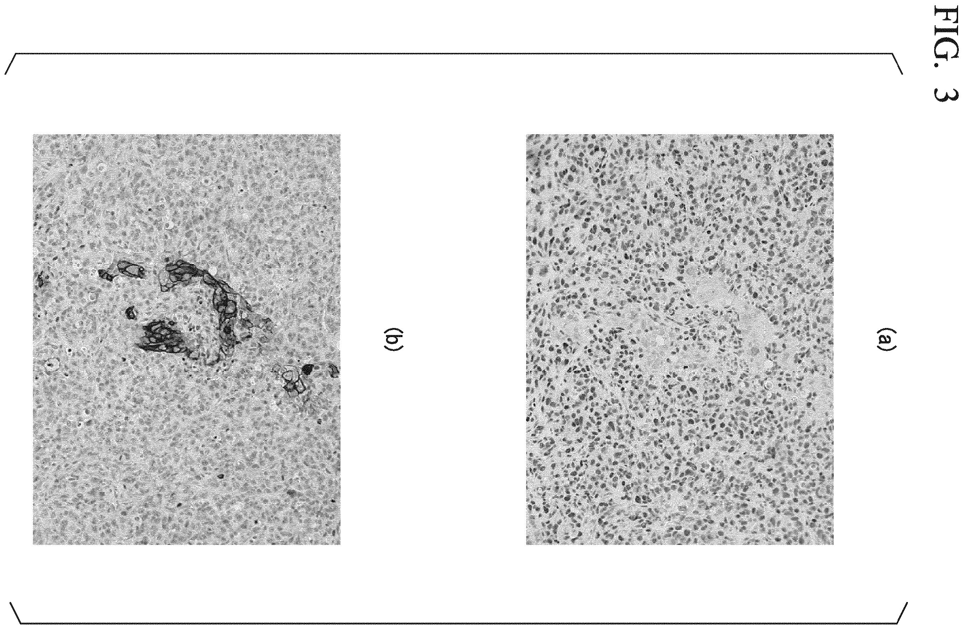

FIG. 3(a) is a photograph showing the results of staining a thin-sectioned tissue sample of a cancer tissue with an anti-ZEB 1 antibody (catalog number "sc-10572", available from Santa Cruz). FIG. 3(b) is a photograph showing the results of staining a thin-sectioned tissue sample having approximately the same field of view as FIG. 3(a) with an anti-CLDNl antibody (catalog number "ab15098", available from Abcam plc).

From the results of Experimental Example 3, it was found that no tumor was formed in the 1937 mouse.

From this result, it was considered that the cancer tissue of the Mix mouse is composed only of ZEB1(+) CLDN1(-) MDA-MB-231 cells and therefore the CLDN1 protein may not be expressed. On the contrary, unexpectedly, the expression of CLDN1 protein as well as ZEB1 protein was confirmed in the cancer tissue of the Mix mouse. In addition, it was shown that the expression of ZEB1 protein and the expression of CLDN1 protein were mutually exclusive, ZEB1(+) cells were CLDN1(-), and CLDN1(+) cells were ZEB1(-).

That is, it was confirmed that ZEB1(+) CLDN1(-) cells and ZEB1(-) CLDN1(+) cells were present in mixture in the cancer tissue of the Mix mouse and therefore heterogeneous cancer tissues were formed.

From the results of Experimental Examples 3 and 4, it was found that a model mouse having a heterogeneous cancer tissue can be constructed by transplanting a mixture of MDA-MB-231 cell line and HCC1937 cell line into an immunodeficient mouse. In addition, since the heterogeneous cancer tissue formed a larger tumor, it was shown that the heterogeneous cancer tissue had higher malignancy than the homogeneous cancer tissue.

### Evaluation of malignancy of heterogeneous cancer tissue

### Experimental Example 5

### Evaluation of lung metastasis

From the Mix mouse and the 231 mouse constructed in Experimental Example 2, lungs were excised 11 weeks after the transplantation of cancer cell lines, fixed with paraformaldehyde, and embedded in paraffin. Subsequently, thin-sectioned tissue samples of the lungs were prepared, stained with hematoxylin/eosin, and observed under a microscope to examine the presence of lung metastatic lesions of cancer.

As a result, there was more formation of lung metastatic lesions in the Mix mouse than the 231 mouse.

This result also indicated that the heterogeneous cancer tissue was more malignant than the homogeneous cancer tissue.

### Experimental Example 6

### Study on therapeutic effects by anticancer drug

Paclitaxel (3 mg/kg) was administered intraperitoneally once a week to Mix mouse and 231 mouse constructed in the same manner as in Experimental Example 2, the tumor diameter was measured with time, and temporal changes in tumor volume were observed (n = 5 in each case). As controls, temporal changes in tumor volume were also observed for the groups (control groups) in which physiological saline was administered intraperitoneally in place of paclitaxel (n = 5 in each case).

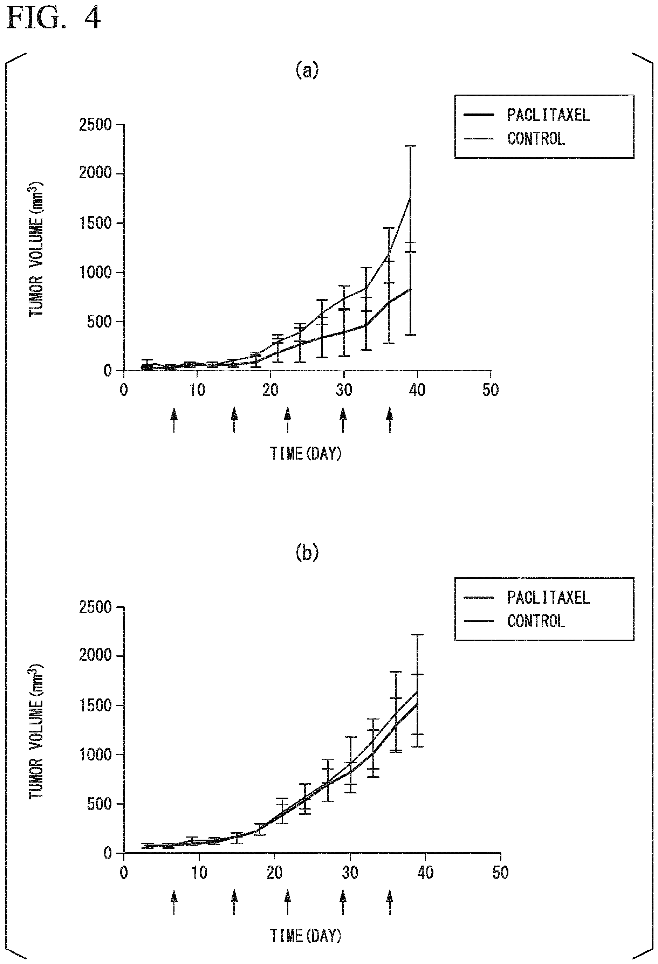

FIG. 4(a) is a graph showing the results of the 231 mouse. FIG. 4(b) is a graph showing the results of the Mix mouse. In the drawing, "Paclitaxel" indicates the results of the paclitaxel administration group and "Control" indicates the results of the control group. As a result, it was found that the cancer tissue of the Mix mouse has higher resistance to paclitaxel than the cancer tissue of the 231 mouse. This result also indicated that the heterogeneous cancer tissue was more malignant than the homogeneous cancer tissue.

### Experimental Example 7

### Study on prognosis of ZEB1(+) CLDN1(+) breast cancer patients

Using the microarray gene expression analysis results of cancer tissue samples derived from 295 breast cancer patients (refer to Chang H. Y. et al., Robustness, scalability, and integration of a wound-response gene expression signature in predicting breast cancer survival., Proc. Natl. Acad. Sci. U.S.A., 102(10), pp 3738-3743, 2005. PMID: 15701700), the prognosis (survival rate) of ZEB1(+) CLDN1(+) breast cancer patients, ZEB1(-) CLDN1(+) breast cancer patients, ZEB1(+) CLDN1(-) breast cancer patients, and ZEB1(-) CLDN1(-) breast cancer patients was studied. Here, the cancer tissue which is ZEB1(+) CLDN1(+) was considered to be a heterogeneous cancer tissue in which ZEB1(+) CLDN1(-) cells and ZEB1(-) CLDN1(+) cells coexist.

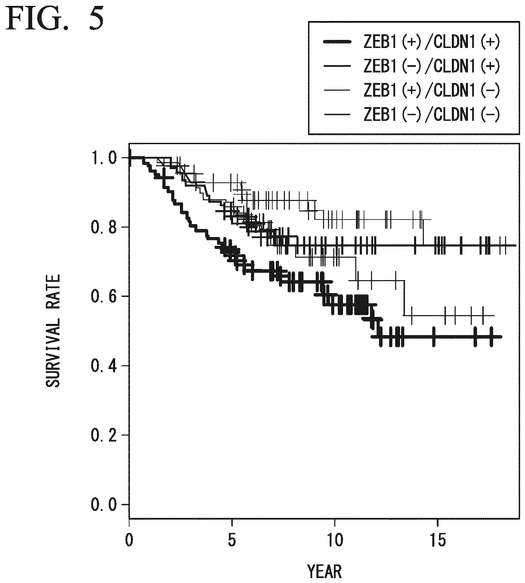

FIG. 5 is a graph showing the study results. As a result, the survival rate of ZEB1(+) CLDN1(+) breast cancer patients was found to be low. This result also indicated that the heterogeneous cancer tissue was more malignant.

As described above, the results of Experimental Examples 4 to 7 indicated that the malignancy was higher in the heterogeneous cancer tissue.

### Search for marker for determining whether or not cancer tissue contains heterogeneous cancer cell population

### Experimental Example 8

### Comprehensive transcriptome analysis

The comprehensive transcriptome analysis was carried out on cancer tissues of Mix mouse, 231 mouse, and 1937 mouse constructed in the same manner as in Experimental Example 2.

First, a cancer tissue was excised from each tumor-bearing mouse and RNA was extracted therefrom. Subsequently, a library was prepared using a kit (trade name "TruSeq RNA Sample Preparation Kit v2", available from Illumina Inc.). Subsequently, sequence analysis was carried out using a next generation sequencer (model "Genome Analyzer IIx", available from Illumina Inc.), and bioinformatics analysis was carried out.

More specifically, the detected base sequence was separated into a human-derived base sequence and a mouse-derived base sequence using the software "Xenome". Subsequently, the obtained base sequence data was mapped to a reference sequence using the software "Tophat". Subsequently, the mapped base sequence data was visualized together with a known gene sequence, and mRNA expression between the respective samples was compared using the software "Avadis".

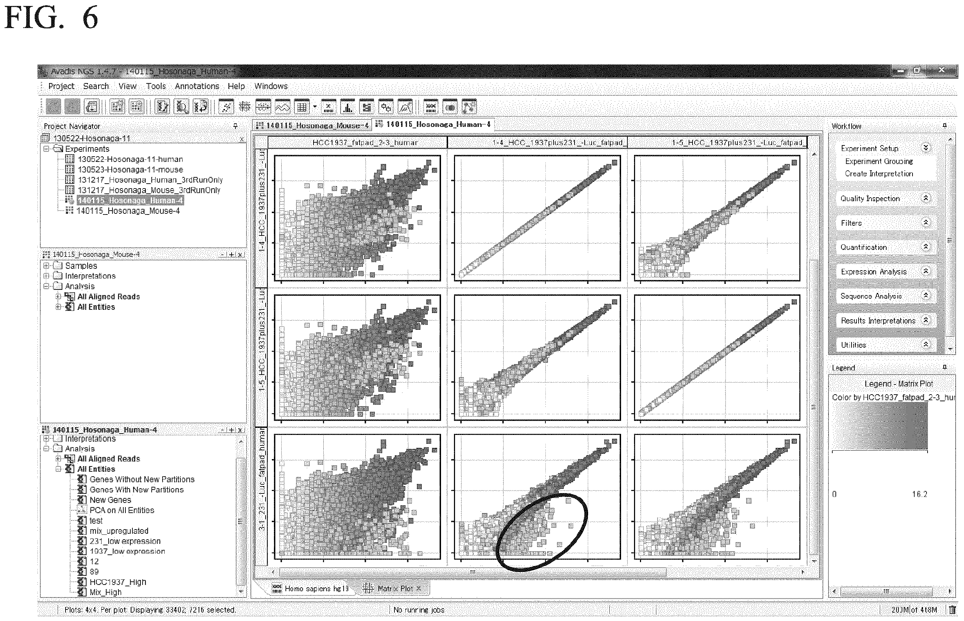

FIG. 6 is a diagram showing the analysis results using the software "Avadis". As a result of analysis using the software "Avadis", the genes shown in the circled area in the middle lower region of FIG. 6 were found as genes that are highly expressed in the cancer tissues of the Mix mouse and are hardly expressed in the cancer tissues of 231 mouse and 1937 mouse. These genes are candidates for a genetic marker for determining whether or not a cancer tissue contains a heterogeneous cancer cell population.

### Experimental Example 9

### Expression analysis of candidate genes by quantitative real-time PCR

The quantitative real-time PCR expression analysis was carried out on the genes found in Experimental Example 8 as a candidate for a genetic marker for determining whether or not a cancer tissue contains a heterogeneous cancer cell population.

First, a cancer tissue was excised from the Mix mouse, 231 mouse and 1937 mouse constructed in the same manner as in Experimental Example 2, and RNA was extracted from a part of the tissue. In addition, a part of the tissue was fixed with paraformaldehyde, embedded in paraffin, and used for the subsequent experiment.

Subsequently, cDNA was synthesized from the extracted RNA, and the expression of the candidate genes was analyzed by quantitative real-time PCR.

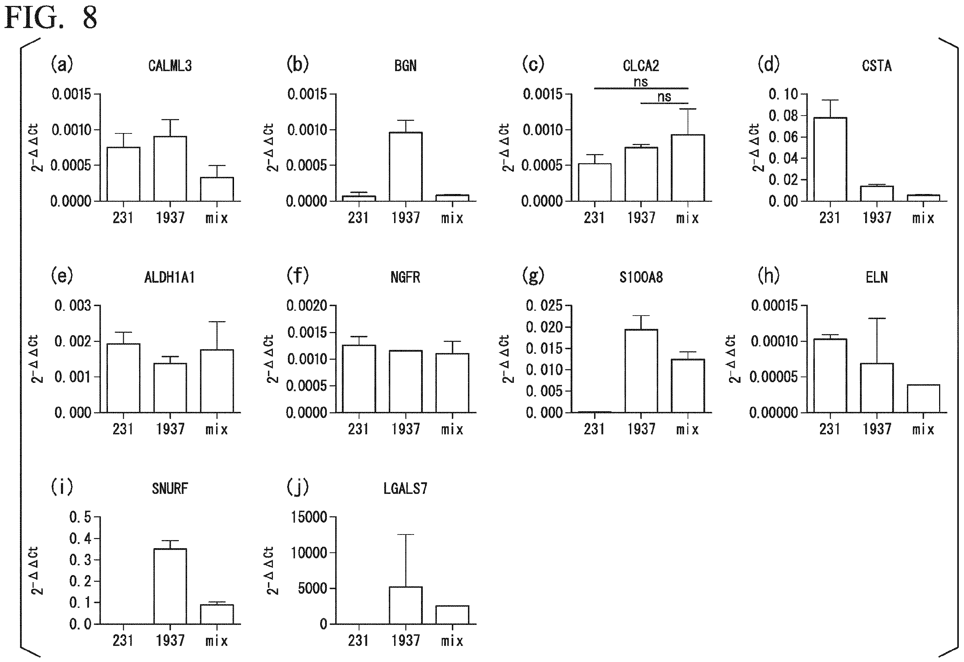

As a result, it was confirmed that CALML3, BGN, CLCA2, CSTA, ALDH1A1, NGFR, S100A8, ELN, SNURF, and LGALS7 genes, which were found to be hardly expressed in the 231 mouse and 1937 mouse in the transcriptome analysis of Example 8, were highly expressed in cancer tissues of the Mix mouse also by quantitative real-time PCR. That is, it was shown that these genes can be used as a genetic marker for determining whether or not a cancer tissue contains a heterogeneous cancer cell population, since the cancer cell population with the transplantation of mixed MDA-MB-231 cells and HCC1937 cells exhibits heterogeneity with detection of high expression of such genes. Among them, CALML3, BGN, CLCA2, CSTA, ALDH1A1, NGFR, ELN, and LGALS7 genes showed high expression in cancer tissues of the Mix mouse, despite the absence of an increase in the expression level thereof (see FIG. 8) even in the case where MDA-MB-231 cells and HCC1937 cells were mixed in *in vitro* experiments to be described later. In the transcriptome analysis, the expression level thereof was greater in comparison with the expression level in cancer tissues of the 231 mouse and 1937 mouse.

Table 1 shows the names and SEQ ID NOs of primers used for quantitative real-time PCR of CALML3, BGN, CLCA2, CSTA, ALDH1A1, NGFR, S100A8, ELN, SNURF, and LGALS7 genes.

**Table 1**

| Gene | Sense primer (SEQ ID NO) | Anti-sense primer (SEQ ID NO) |
|---|---|---|
| CALML3 | CALML3-F (5) | CALML3-R (6) |
| BGN | BGN-F (7) | BGN-R (8) |
| CLCA2 | CLCA2-F (9) | CLCA2-R (10) |
| CSTA | CSTA-F (11) | CSTA-R (12) |
| ALDH1A1 | ALDH1A1-F (13) | ALDH1A1-R (14) |
| NGFR | NGFR-F (15) | NGFR-R (16) |
| S100A8 | S100A8-F (17) | S100A8-R (18) |
| ELN | ELN-F (19) | ELN-R (20) |
| SNURF | SNURF-F (21) | SNURF-R (22) |
| LGALS7 | LGALS7/7B-F (23) | LGALS7/7B-R (24) |

### Experimental Example 10

### Expression analysis of candidate genes by immunostaining

For the genes found in Experimental Example 8 as a candidate for a genetic marker for determining whether or not a cancer tissue contains a heterogeneous cancer cell population, the expression of those genes was studied by immunostaining.

More specifically, samples of cancer tissues of the Mix mouse and 231 mouse constructed in Experimental Example 9 were thin-sectioned to prepare tissue section samples which were then subjected to hematoxylin/eosin staining and immunostaining.

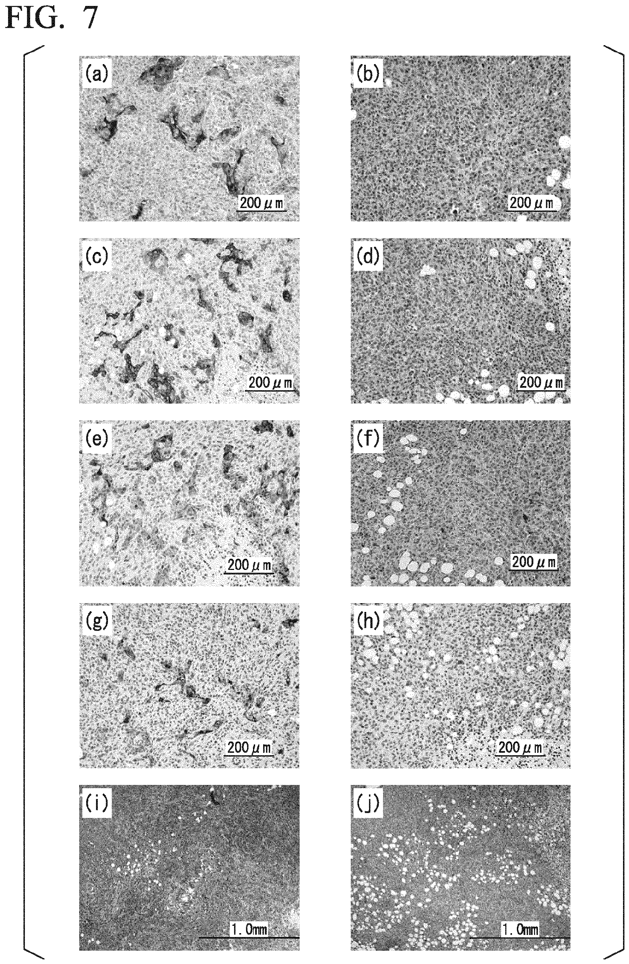

FIGS. 7(a) to 7(j) are photographs showing the representative results of immunostaining. FIG. 7(a) is a photograph showing the results of staining the cancer tissue sample of the Mix mouse with an anti-CALML3 antibody (catalog number "GTX114954", available from Gene Tex Inc.). FIG. 7(b) is a photograph showing the results of staining the cancer tissue sample of the 231 mouse with an anti-CALML3 antibody (same as above), as a control.

FIG. 7(c) is a photograph showing the results of staining the cancer tissue sample of the Mix mouse with an anti-CLCA2 antibody (catalog number "NBP2-33482", available from Novus Biologicals, LLC). FIG. 7(d) is a photograph showing the results of staining the cancer tissue sample of the 231 mouse with an anti-CLCA2 antibody (same as above), as a control. FIG. 7(e) is a photograph showing the results of staining the cancer tissue sample of the Mix mouse with an anti-CSTA antibody (catalog number "HPA001031", available from ATRAS Antibodies AB). FIG. 7(f) is a photograph showing the results of staining the cancer tissue sample of the 231 mouse with an anti-CSTA antibody (same as above), as a control. FIG. 7(g) is a photograph showing the results of staining the cancer tissue sample of the Mix mouse with an anti-LGALS7 antibody (catalog number "HPA001549", available from ATRAS Antibodies AB). FIG. 7(h) is a photograph showing the results of staining the cancer tissue sample of the 231 mouse with an anti-LGALS7 antibody (same as above), as a control. FIG. 7(i) is a photograph showing the results of staining the cancer tissue sample of the Mix mouse with an anti-S100A8 antibody (catalog number "NBP2-25269", available from Novus Biologicals, LLC). FIG. 7(j) is a photograph showing the results of staining the cancer tissue sample of the 231 mouse with an anti-S100A8 antibody (same as above), as a control.

As a result, it was confirmed that the expression of CALML3, CLCA2, CSTA, LGALS7 and S100A8 proteins was high in cancer tissues of the Mix mouse and was hardly observed in cancer tissues of the 231 mouse.

### Experimental Example 11

### Expression analysis of candidate genes by quantitative real-time PCR using in vitro samples

Using *in vitro* samples, the quantitative real-time PCR expression analysis was carried out on the genes found in Experimental Example 8 as a candidate for a genetic marker for determining whether or not a cancer tissue contains a heterogeneous cancer cell population.

First, 231 cells, 1937 cells, and cells in which 231 cells and 1937 cells were mixed in a ratio of 1:1 were respectively cultured in a culture dish for 5 days to prepare *in vitro* samples. Subsequently, RNA was extracted from each cell sample. Subsequently, cDNA was synthesized from the extracted RNA, and the expression of the candidate genes was analyzed by quantitative real-time PCR. The quantitative real-time PCR was carried out in the same manner as in Experimental Example 9.

FIG. 8 is a graph showing the results of quantitative real-time PCR. In FIG. 8, "231" indicates that it is a result of 231 cells, "1937" indicates that it is a result of 1937 cells, and "mix" indicates that it is a result of mixed cells of 231 cells and 1937 cells in a ratio of 1:1.

As a result, high expression of CALML3, BGN, CLCA2, CSTA, ALDHA1, NGFR, S100A8, ELN, SNURF, and LGALS7 genes as seen in *in vivo* samples from the transplanted cancer cell population obtained in Experimental Examples 9 and 10 could not be confirmed in the sample in which 231 cells and 1937 cells had been mixed *in vitro.*

From this result, it was found that a genetic marker for determining whether or not a cancer tissue contains a heterogeneous cancer cell population is not highly expressed merely by mixing and culturing cancer cells *in vitro,* and is highly expressed only in the case where a heterogeneous cancer cell population is formed *in vivo.*

### Experimental Example 12

### Immunostaining of breast cancer surgical specimens

For the genes found in Experimental Example 8 as a candidate for a genetic marker for determining whether or not a cancer tissue contains a heterogeneous cancer cell population, the expression of such genes was studied by immunostaining of clinical specimens.

More specifically, immunohistological staining was carried out using formalin-fixed, paraffin-embedded thin-sectioned tissue samples of tissues obtained by surgery of breast cancer patients who provided informed consent, and the expression of CALML3, CLCA2, CSTA and LGALS7 proteins was studied.

As a result, staining of CALML3, CLCA2, CSTA and LGALS7 proteins was confirmed, and it was confirmed that the expression of these marker proteins could be detected in human breast cancer surgical specimens.

This result shows that genetic markers, cDNA markers, and protein markers for determining whether or not a cancer tissue contains a heterogeneous cancer cell population can be practically used in human samples.

### Industrial Applicability

According to the present invention, it is possible to use the genetic marker described herein for determining whether or not a cancer tissue contains a heterogeneous cancer cell population. Further, it is possible to determine whether or not a cancer tissue contains a heterogeneous cancer cell population by using the protein marker, kit, or method described herein.

## Claims

1. Use of a genetic marker being selected from the group consisting of Calmodulin-like protein 3 (CALML3), Biglycan (BGN), Chloride channel accessory 2 (CLCA2), Cystatin A (CSTA), Aldehyde dehydrogenase 1 family, member A1 (ALDH1A1), Nerve growth factor receptor (NGFR), S100-calcium-binding protein A8 (S100A8), Elastin (ELN), SNRPN upstream reading frame (SNURF), and Galectin-7 (LGALS7) for determining whether or not a cancer tissue contains a heterogeneous cancer cell population.

2. Use of a protein marker being encoded by a gene selected from the group consisting of CALML3, BGN, CLCA2, CSTA, ALDH1A1, NGFR, S100A8, ELN, SNURF, and LGALS7, for determining whether or not a cancer tissue contains a heterogeneous cancer cell population.

3. Use of a gene selected from the group consisting of CALML3, BGN, CLCA2, CSTA, NGFR, S100A8, ELN, SNURF, and LGALS7, or a protein encoded by the gene, for detecting a heterogeneous cancer cell population to predict a prognosis of a cancer patient.

4. Use of a kit comprising:
a primer set for amplifying cDNA of at least one gene selected from the group consisting of CALML3, BGN, CLCA2, CSTA, ALDH1A1, NGFR, S100A8, ELN, SNURF, and LGALS7;
a probe that specifically hybridizes to mRNA of at least one gene selected from the group consisting of CALML3, BGN, CLCA2, CSTA, ALDH1A1, NGFR, S100A8, ELN, SNURF, and LGALS7; or
a specific binding substance to a protein encoded by at least one gene selected from the group consisting of CALML3, BGN, CLCA2, CSTA, ALDH1A1, NGFR, S100A8, ELN, SNURF, and LGALS7,
for determining whether or not a cancer tissue contains a heterogeneous cancer cell population.

5. Use of a kit comprising:
a primer set for amplifying cDNA of at least one gene selected from the group consisting of CALML3, BGN, CLCA2, CSTA, NGFR, S100A8, ELN, SNURF, and LGALS7;
a probe that specifically hybridizes to mRNA of at least one gene selected from the group consisting of CALML3, BGN, CLCA2, CSTA, NGFR, S100A8, ELN, SNURF, and LGALS7; or
a specific binding substance to a protein encoded by at least one gene selected from the group consisting of CALML3, BGN, CLCA2, CSTA, NGFR, S100A8, ELN, SNURF, and LGALS7,
for detecting a heterogeneous cancer cell population to predict a prognosis of a cancer patient.

6. A method for determining whether or not a cancer tissue sample contains a heterogeneous cancer cell population, comprising:
a detection step of detecting the expression of at least one gene selected from the group consisting of CALML3, BGN, CLCA2, CSTA, ALDH1A1, NGFR, S100A8, ELN, SNURF, and LGALS7 in a cancer tissue sample; and
a step of determining that the cancer tissue sample contains a heterogeneous cancer cell population in the case where the expression of the gene is detected.

7. The determination method according to Claim 6, wherein
(i) the detection step is carried out by detecting mRNA of the gene; or
(ii) the detection step is carried out by detecting a protein encoded by the gene.

8. The determination method according to Claim 6 or 7, wherein the cancer tissue sample is derived from breast cancer, melanoma, lung cancer, or pancreatic cancer.

9. The determination method according to Claim 8, wherein the breast cancer is an estrogen receptor(-) progesterone receptor(-) HER2(-) breast cancer.

10. The determination method according to any one of Claims 6 to 9, wherein the heterogeneous cancer cell population includes ZEB1(+) CLDN1(-) cells and ZEB1(-) CLDN1(+) cells.

11. A method for detecting a heterogeneous cancer cell population to predict a prognosis of cancer, comprising:
a detection step of detecting the expression of at least one gene selected from the group consisting of CALML3, BGN, CLCA2, CSTA, NGFR, S100A8, ELN, SNURF, and LGALS7 in a cancer tissue sample; and
a step of determining that a patient from whom the cancer tissue sample is derived has a poor prognosis in the case where the expression of the gene is detected.

12. A method for preparing a heterogeneous cancer cell population expressing a gene selected from the group consisting of CALML3, BGN, CLCA2, CSTA, ALDH1A1, NGFR, S100A8, ELN, SNURF, and LGALS7, or a protein encoded by the gene, comprising the steps of mixing a cell line that does not form a tumor upon single transplantation with a cell line that forms a tumor even upon single transplantation and transplanting such a cell line mixture into a nude mouse.

13. The method for preparing a heterogeneous cancer cell population according to Claim 12, wherein the heterogeneous cancer cell population is isolated from the nude mouse.

## Patentansprüche

1. Verwendung eines genetischen Markers, ausgewählt aus der Gruppe, bestehend aus Calmodulin-ähnlichem Protein 3 (CALML3), Biglycan (BGN), Chlorid-Kanal-Accessory 2 (CLCA2), Cystatin A (CSTA), Mitglied A1 der Aldehyddehydrogenase 1-Familie (ALDH1A1), Nervenwachstumsfaktor-Rezeptor (NGFR), S100-Calcium-bindendem Protein A8 (S100A8), Elastin (ELN), SNRPN stromaufwärts gelegenem Leserahmen (SNURF) und Galectin-7 (LGALS7), zum Bestimmen, ob ein Krebsgewebe eine heterogene Krebszellpopulation enthält oder nicht.

2. Verwendung eines Proteinmarkers, der von einem Gen kodiert wird, das aus der Gruppe ausgewählt ist, die aus CALML3, BGN, CLCA2, CSTA, ALDH1A1, NGFR, S100A8, ELN, SNURF und LGALS7 besteht, zum Bestimmen, ob ein Krebsgewebe eine heterogene Krebszellpopulation enthält oder nicht.

3. Verwendung eines Gens, ausgewählt aus der Gruppe, bestehend aus CALML3, BGN, CLCA2, CSTA, NGFR, S100A8, ELN, SNURF und LGALS7, oder eines durch das Gen kodierten Proteins zum Nachweisen einer heterogenen Krebszellpopulation, um eine Prognose eines Krebspatienten zu prognostizieren.

4. Verwendung eines Kits, umfassend:
einen Primersatz zum Amplifizieren von cDNA mindestens eines Gens, ausgewählt aus der Gruppe, bestehend aus CALML3, BGN, CLCA2, CSTA, ALDH1A1, NGFR, S100A8, ELN, SNURF und LGALS7;
eine Sonde, die spezifisch an mRNA mindestens eines Gens, ausgewählt aus der Gruppe, bestehend aus CALML3, BGN, CLCA2, CSTA, ALDH1A1, NGFR, S100A8, ELN, SNURF und LGALS7, hybridisiert; oder
eine spezifische Bindungssubstanz an ein Protein, das von mindestens einem Gen, ausgewählt aus der Gruppe, bestehend aus CALML3, BGN, CLCA2, CSTA, ALDH1A1, NGFR, S100A8, ELN, SNURF und LGALS7, kodiert wird,
zum Bestimmen, ob ein Krebsgewebe eine heterogene Krebszellpopulation enthält oder nicht.

5. Verwendung eines Kits, umfassend:
einen Primersatz zum Amplifizieren von cDNA mindestens eines Gens, ausgewählt aus der Gruppe, bestehend aus CALML3, BGN, CLCA2, CSTA, NGFR, S100A8, ELN, SNURF und LGALS7;
eine Sonde, die spezifisch an mRNA mindestens eines Gens, ausgewählt aus der Gruppe, bestehend aus CALML3, BGN, CLCA2, CSTA, NGFR, S100A8, ELN, SNURF und LGALS7, hybridisiert; oder
eine spezifische Bindungssubstanz an ein Protein, das von mindestens einem Gen, ausgewählt aus der Gruppe, bestehend aus CALML3, BGN, CLCA2, CSTA, NGFR, S100A8, ELN, SNURF und LGALS7, kodiert wird,
zum Nachweisen einer heterogenen Krebszellpopulation, um eine Prognose eines Krebspatienten zu prognostizieren.

6. Verfahren zum Bestimmen, ob eine Krebsgewebeprobe eine heterogene Krebszellpopulation enthält oder nicht, umfassend:
einen Nachweisschritt eines Nachweisens der Expression mindestens eines Gens, ausgewählt aus der Gruppe, bestehend aus CALML3, BGN, CLCA2, CSTA, ALDH1A1, NGFR, S100A8, ELN, SNURF und LGALS7, in einer Krebsgewebeprobe; und
einen Schritt eines Bestimmens, dass die Krebsgewebeprobe eine heterogene Krebszellpopulation enthält in dem Fall, bei dem die Expression des Gens nachgewiesen wird.

7. Bestimmungsverfahren gemäß Anspruch 6, wobei
(i) der Nachweisschritt durch Nachweisen von mRNA des Gens erfolgt; oder
(ii) der Nachweisschritt durch Nachweisen eines durch das Gen kodierten Proteins erfolgt.

8. Bestimmungsverfahren gemäß Anspruch 6 oder 7, wobei die Krebsgewebeprobe von Brustkrebs, Melanom, Lungenkrebs oder Pankreaskrebs abstammt.

9. Bestimmungsverfahren gemäß Anspruch 8, wobei der Brustkrebs ein Östrogen-Rezeptor(-)-Progesteron-Rezeptor(-)-HER2(-)-Brustkrebs ist.

10. Bestimmungsverfahren gemäß einem jeglichen der Ansprüche 6 bis 9, wobei die heterogene Krebszellpopulation ZEB1(+)-CLDN1 (-)-Zellen und ZEB1 (-)-CLDN1(+)-Zellen beinhaltet.

11. Verfahren zum Nachweisen einer heterogenen Krebszellpopulation, um eine Prognose eines Krebspatienten zu prognostizieren, umfassend:
einen Nachweisschritt eines Nachweisens der Expression mindestens eines Gens, ausgewählt aus der Gruppe, bestehend aus CALML3, BGN, CLCA2, CSTA, NGFR, S100A8, ELN, SNURF und LGALS7, in einer Krebsgewebeprobe; und
einen Schritt eines Bestimmens, dass ein Patient, von dem die Krebsgewebeprobe abstammt, eine schlechte Prognose aufweist in dem Fall, bei dem die Expression des Gens nachgewiesen wird.

12. Verfahren zum Herstellen einer heterogenen Krebszellpopulation, die ein Gen, ausgewählt aus der Gruppe, bestehend aus CALML3, BGN, CLCA2, CSTA, ALDH1A1, NGFR, S100A8, ELN, SNURF und LGALS7, oder ein durch das Gen kodiertes Protein exprimiert, umfassend die Schritte eines Mischens einer Zellline, die keinen Tumor nach einer einzigen Transplantation bildet, mit einer Zellline, die einen Tumor sogar nach einer einzigen Transplantation bildet, und eines Transplantierens eines solchen Zellliniengemisches in eine Nacktmaus.

13. Verfahren zum Herstellen einer heterogenen Krebszellpopulation gemäß Anspruch 12, wobei die heterogene Krebszellpopulation aus der Nacktmaus isoliert wird.

## Revendications

1. Utilisation d'un marqueur génétique choisi dans le groupe constitué par la protéine 3 analogue à la calmoduline (CALML3), le Biglycane (BGN), le canal Chlorure accessoire 2 (CLCA2), la Cystatine A (CSTA), le membre Al de la famille de l'aldéhyde déshydrogénase 1 (ALDH1A1), le récepteur du facteur de croissance des nerfs (NGFR), la protéine A8 de liaison au calcium SI100 (S100A8), l'Elastine (ELN), le cadre de lecture en amont SNRPN (SNURF) et la Galectine-7 (LGALS7) pour déterminer si un tissu cancéreux contient ou non une population hétérogène de cellules cancéreuses.

2. Utilisation d'un marqueur protéique codé par un gène choisi dans le groupe constitué par CALML3, BGN, CLCA2, CSTA, ALDH1A1, NGFR, S100A8, ELN, SNURF et LGALS7, pour déterminer si un tissu cancéreux contient ou non une population hétérogène de cellules cancéreuses.

3. Utilisation d'un gène choisi dans le groupe constitué par CALML3, BGN, CLCA2, CSTA, NGFR, S100A8, ELN, SNURF et LGALS7, ou d'une protéine codée par le gène, pour détecter une population hétérogène de cellules cancéreuses afin de prédire le pronostic d'un patient cancéreux.

4. Utilisation d'un kit comprenant :
un ensemble d'amorces pour amplifier l'ADNc d'au moins un gène choisi dans le groupe constitué par CALML3, BGN, CLCA2, CSTA, ALDH1A1, NGFR, S100A8, ELN, SNURF et LGALS7 ;
une sonde qui s'hybride spécifiquement à l'ARNm d'au moins un gène choisi dans le groupe constitué par CALML3, BGN, CLCA2, CSTA, ALDH1A1, NGFR, S100A8, ELN, SNURF et LGALS7 ; ou
une substance de liaison spécifique à une protéine codée par au moins un gène choisi dans le groupe constitué par CALML3, BGN, CLCA2, CSTA, ALDH1A1, NGFR, S100A8, ELN, SNURF et LGALS7,
pour déterminer si un tissu cancéreux contient ou non une population hétérogène de cellules cancéreuses.

5. Utilisation d'un kit comprenant :
un ensemble d'amorces pour amplifier l'ADNc d'au moins un gène choisi dans le groupe constitué par CALML3, BGN, CLCA2, CSTA, NGFR, S100A8, ELN, SNURF et LGALS7 ;
une sonde qui s'hybride spécifiquement à l'ARNm d'au moins un gène choisi dans le groupe constitué par CALML3, BGN, CLCA2, CSTA, NGFR, S100A8, ELN, SNURF et LGALS7 ; ou
une substance de liaison spécifique à une protéine codée par au moins un gène choisi dans le groupe constitué par CALML3, BGN, CLCA2, CSTA, NGFR, S100A8, ELN, SNURF et LGALS7,
pour détecter une population hétérogène de cellules cancéreuses afin de prédire le pronostic d'un patient cancéreux.

6. Procédé pour déterminer si un échantillon de tissu cancéreux contient ou non une population hétérogène de cellules cancéreuses, comprenant :
une étape de détection consistant à détecter l'expression d'au moins un gène choisi dans le groupe constitué par CALML3, BGN, CLCA2, CSTA, ALDH1A1, NGFR, S100A8, ELN, SNURF et LGALS7 dans un échantillon de tissu cancéreux ; et
une étape consistant à déterminer que l'échantillon de tissu cancéreux contient une population hétérogène de cellules cancéreuses dans le cas où l'expression du gène est détectée.

7. Procédé de détermination selon la revendication 6, dans lequel
(i) l'étape de détection est effectuée en détectant l'ARNm du gène ; ou
(ii) l'étape de détection est effectuée en détectant une protéine codée par le gène.

8. Procédé de détermination selon la revendication 6 ou 7, dans lequel l'échantillon de tissu cancéreux est dérivé d'un cancer du sein, d'un mélanome, d'un cancer du poumon ou d'un cancer du pancréas.

9. Procédé de détermination selon la revendication 8, dans lequel le cancer du sein est un cancer du sein à récepteur d'œstrogène(-) récepteur de progestérone(-) HER2(-).

10. Procédé de détermination selon l'une quelconque des revendications 6 à 9, dans lequel la population hétérogène de cellules cancéreuses comprend des cellules ZEB1(+) CLDN1(-) et des cellules ZEB1(-) CLDN1(+).

11. Procédé pour détecter une population hétérogène de cellules cancéreuses afin de prédire un pronostic de cancer, comprenant :
une étape de détection consistant à détecter l'expression d'au moins un gène choisi dans le groupe constitué de CALML3, BGN, CLCA2, CSTA, NGFR, S100A8, ELN, SNURF et LGALS7 dans un échantillon de tissu cancéreux ; et
une étape consistant à déterminer qu'un patient duquel provient l'échantillon de tissu cancéreux a un mauvais pronostic dans le cas où l'expression du gène est détectée.

12. Procédé de préparation d'une population hétérogène de cellules cancéreuses exprimant un gène choisi dans le groupe constitué par CALML3, BGN, CLCA2, CSTA, ALDH1A1, NGFR, S100A8, ELN, SNURF et LGALS7, ou une protéine codée par le gène, comprenant les étapes consistant à mélanger une lignée cellulaire qui ne forme pas de tumeur après une seule transplantation avec une lignée cellulaire qui forme une tumeur même après une seule transplantation et à transplanter un tel mélange de lignées cellulaires dans une souris nue.

13. Procédé de préparation d'une population hétérogène de cellules cancéreuses selon la revendication 12, dans lequel la population hétérogène de cellules cancéreuses est isolée de la souris nue.
